(19) Europäisches Patentamt
European Patent Office
Office européen des brevets

(11) Publication number: 0 136 586
B1

(12) EUROPEAN PATENT SPECIFICATION

(45) Date of publication of patent specification: 01.03.89

(51) Int. Cl.[4]: C 07 J 5/00, C 07 J 7/00, C 07 J 31/00, A 61 K 31/57

(21) Application number: 84110572.9

(22) Date of filing: 05.09.84

# (54) Novel corticoid derivatives and process for production thereof.

(30) Priority: 07.09.83 JP 164772/83
12.06.84 JP 120439/84

(43) Date of publication of application:
10.04.85 Bulletin 85/15

(45) Publication of the grant of the patent:
01.03.89 Bulletin 89/09

(84) Designated Contracting States:
AT BE CH DE FR GB IT LI NL SE

(56) References cited:
EP-A-0 000 742
EP-A-0 004 975
EP-A-0 072 546
DE-B-1 518 179
LU-A- 68 212
US-A-3 558 675

(73) Proprietor: MITSUBISHI KASEI CORPORATION
5-2, Marunouchi 2-chome Chiyoda-ku
Tokyo 100 (JP)

(72) Inventor: Nitta, Issei
4-13-22 Narusedai
Machida-shi Tokyo (JP)
Inventor: Nakao, Kenichiro
4-18-24 Soshigaya
Setagaya-ku Tokyo (JP)
Inventor: Miyake, Motoyoshi
2-2-4 Eifuku
Suginami-ku Tokyo (JP)
Inventor: Maruyama, Akira
23-4 Tana-machi Midori-ku
Yokohama-shi Kanagawa-ken (JP)
Inventor: Takashima, Junko
3-23-1 Tsuchihashi Miyamae-ku
Kawasaki-shi Kanagawa-ken (JP)
Inventor: Ueno, Hiroaki Mitsubishi Kasei Aoba-Ryo
5-1 Tsutsujigaoka Midori-ku
Yokohama-shi Kanagawa-ken (JP)

(74) Representative: Patentanwälte TER MEER - MÜLLER - STEINMEISTER
Mauerkircherstrasse 45
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 136 586 B1

Courier Press, Leamington Spa, England.

**Description**

Background of the Invention

This invention relates to novel corticoid derivatives. Corticoid derivatives have an anti-inflammatory activity and are useful as pharmaceuticals. As corticoid derivatives, 6-oxygenated corticoids are disclosed in Japanese Unexamined Patent Publication No. 73765/79 and corticoid 17α-carbonates are disclosed in Japanese Unexamined Patent Publication No. 36248/79. However, novel derivatives that have more efficacy as pharmaceuticals are always in demand.

EP—A—4975 discloses corticoid 17-alkyl carbonates having an anti-inflammatory activity, which comprise as substituent Y in ring B of the steroid system hydrogen, fluorine or chlorine.

DE—A—1 518 719 describes 17(β-carbomethoxy) proprionate of $\Delta^4$-pregnen-17α-21-diol-3,20-dione useful as a raw material for the synthesis of 17α-hydroxy-progesteron-β-carbomethoxy-proprionate. This compound does not have an 11-oxo or 11β-hydroxy group.

EP—A—72 546 discloses hydrocortisone derivatives having an anti-inflammatory activity. Substituent Y on the ring B of the steroid residue comprises hydrogen, chlorine, fluorine or methyl.

The present inventors have synthesized novel corticoid derivatives and found that they have a strong topical anti-inflammatory activity and extremely weak systemic adverse reactions and that they are useful for the treatment of acute and chronic eczema, eczema seborrhoicorum, contact dermatitis, atopic dermatitis, psoriasis, asthma, allergic rhinitis, vasomotor rhinitis, hemorrhoids, auditory disorder, ocular disorder, stomatitis, etc. Thus, the present invention has been completed.

Summary of the Invention

The novel corticoid derivatives of the present invention is represented by the general formula (I):

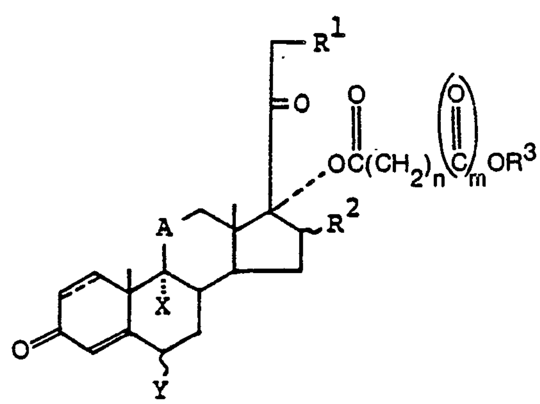

(I)

[wherein A is a group

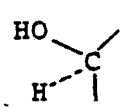 or 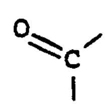 ;

X is a hydrogen atom or halogen atom; Y is a hydrogen atom, oxo group or α- or β-halogen atom, hydroxyl group or alkyl group having 1 to 10 carbon atoms; m is 0 or 1; n is 0 or an integer of 2 to 5; $R^1$ is a hydroxyl group, halogen atom, a group represented by the formula (II):

$$—OSO_2R^4 \qquad (III)$$

(wherein $R^4$ is an alkyl group or halogenated alkyl group having 1 to 10 carbon atoms), or a group represented by the formula (IV):

$$—OCOR^5 \qquad (IV)$$

(wherein $R^5$ is an alkyl group or halogenated alkyl group having 1 to 10 carbon atoms); $R^2$ is a hydrogen atom or alkyl group having 1 to 10 carbon atoms at the α- or β-position; $R^3$ is an alkyl group having 1 to 10 carbon atoms; and the bond between $C_1$ and $C_2$ is a single bond or double bond, provided where m is 0, n is 0 and Y is an oxo group and where m is 1, n is an integer of 2 to 5].

Detailed Description of the Invention

In explaining the present invention in detail, corticoid derivatives of the present invention is represented by the above formula (I).

In the above formula, X is a hydrogen atom or halogen atom selected from fluorine, chlorine, bromine and iodine and fluorine and chlorine are particularly preferred.

Y is a hydrogen atom, oxo group, or α- or β-halogen atom, hydroxyl group or alkyl group having 1 to 10 carbon atoms such as methyl, ethyl, propyl and butyl groups, etc. and a hydrogen atom and oxo group are particularly preferred.

m is 0 or 1. n is 0 or an integer of 2 to 5 and 0, 2 and 3 are particularly preferred. Where m is 0, n is also 0 and Y is an oxo group and where m is 1, n is an integer of 2 to 5.

$R^1$ is a hydroxyl group, halogen atom selected from fluorine, chlorine, bromine and iodine, a group represented by the formula (III):

$$—OSO_2R^4 \qquad \text{(III)}$$

(wherein $R^4$ is an alkyl group or halogenated alkyl group having 1 to 10 carbon atoms), or a group represented by the formula (IV):

$$—OCOR^5 \qquad \text{(IV)}$$

(wherein $R^5$ is an alkyl group or halogenated alkyl group having 1 to 10 carbon atoms), and a halogen atom is particularly preferred.

As the examples of $R^4$ and $R^5$, alkyl groups having 1 to 10 carbon atoms such as methyl, ethyl, propyl and butyl groups, etc. and halogenated alkyl groups having 1 to 10 carbon atoms such as trifluoromethyl, fluoromethyl, chloromethyl, chloroethyl and chloropropyl groups, etc. are mentioned.

$R^3$ is an alkyl group having 1 to 10 carbon atoms such as methyl, ethyl, propyl and butyl groups, etc. and methyl and ethyl groups are particularly preferred.

$R^2$ is a hydrogen atom or alkyl group having 1 to 10 carbon atoms such as methyl, ethyl, propyl and butyl groups, etc. and either α-position or β-position is acceptable. Particularly α-methyl and β-methyl groups are preferred.

As specific examples of corticoid derivatives of the present invention, there may be mentioned 9α-fluoro-16β-methyl-1,4-pregnadiene-11β,17α,21-triol-3,6,20-trione 17α-methyl carbonate; 9α-fluoro-16β-methyl-1,4-pregnadiene-11β,17α,21-triol-3,6,20-trione 17α-methyl carbonate 21-propionate; 9α-fluoro-16β-methyl-1,4-pregnadiene-11β,17α,21-triol-3,6,20-trione 17α-methylcarbonate 21-trifluoromethanesulfonate; 21-chloro-9α-fluoro-16β-methyl-1,4-pregnadiene-11β,17α-diol-3,6,20-trione 17α-methylcarbonate; 9α-fluoro-16β-methyl-1,4-pregnadiene-11β,17α,21-triol-3,6,20-trione 17α-ethylcarbonate; 9α-fluoro-16α-methyl-1,4-pregnadiene-11β,17α,21-triol-3,6,20-trione 17α-methylcarbonate; 9α-fluoro-16β-methyl-4-pregnene-11β,17α,21-triol-3,6,20-trione 17α-methylcarbonate; 21-chloro-9α-fluoro-16β-methyl-1,4-pregnadiene-11β,17α-diol-3,6,20-trione 17α-ethylcarbonate; 9α-fluoro-16β-methyl-1,4-pregnadiene-11β,17α,21-triol-3,6,20-trione 17α-propylcarbonate; 21-chloro-9α-fluoro-16β-methyl-1,4-pregnadiene-11β,17α-diol-3,6,20-trione 17α-propylcarbonate; 21-chloro-9α-fluoro-16α-methyl-1,4-pregnadiene-11β,17α-diol-3,6,20-trione 17α-methylcarbonate; 21-chloro-9α-fluoro-16β-methyl-4-pregnene-17α-ol-3,6,11,20-tetraone 17α-methylcarbonate; 21-chloro-9α-fluoro-16β-methyl-1,4-pregnadiene-17α-ol-3,6,11,20-tetraone 17α-ethylcarbonate; 9α,21-dichloro-16β-methyl-1,4-pregnadiene-11β,17α-diol-3,6,20-trione 17α-methylcarbonate; 9α-fluoro-16β-methyl-pregna-1,4-diene-11β,17α,21-triol-3,6,20-trione 17α-methylcarbonate 21-methanesulfonate; 9α-fluoro-16β-methyl-pregna-1,4-diene-11β,17α,21-triol-3,6,20-trione 17α-methylcarbonate 21-chlorobutylate; 9α-fluoro-21-chloro-pregna-1,4-diene-11β,17α-diol-3,6,21-trione 17α-ethylcarbonate; 9α,21-difluoro-16β-methyl-1,4-pregnadiene-11β,17α-diol-3,6,20-trione 17α-ethylcarbonate; 9α-fluoro-21-iodo-16β-methyl-1,4-pregnadiene-11β,17α-diol-3,6,20-trione 17α-ethylcarbonate; 4-pregnene-11β,17α,21-triol-3,20-dione 17α-(β-methoxycarbonyl propionate); 4-pregnene-17α,21-diol-3,11,20-trione 17α-(β-methoxycarbonyl propionate); 4-pregnene-11β,17α,21-triol-3,20-dione 17α-(β-methoxycarbonyl propionate)21-propionate; 9α-fluoro-16β-methyl-1,4-pregnadiene-11β,17α,21-triol-3,20-dione 17α-(β-methoxycarbonyl propionate); 9α-fluoro-16α-methyl-1,4-pregnadiene-11β,17α,21-triol-3,20-dione 17α-(β-methoxycarbonyl propionate); 9α-fluoro-16β-methyl-1,4-pregnadiene-11β,17α,21-triol-3,20-dione 17α-(β-methoxycarbonyl propionate) 21-propionate; 9α-fluoro-16β-methyl-1,4-pregnadiene-11β,17α,21-triol-3,20-dione 17α-(β-methoxycarbonyl propionate)21-methanesulfonate; 9α-fluoro-16β-methyl-1,4-pregnadiene-11β,17α,21-triol-3,20-dione 17α-(β-ethoxycarbonyl propionate); 21-chloro-9α-fluoro-1,4-pregnadiene-11β,17α-diol-3,20-dione 17α-(β-methoxycarbonyl propionate); 21-chloro-9α-fluoro-16β-methyl-1,4-pregnadiene-11β,17α-diol-3,20-dione 17α-(β-methoxycarbonyl propionate); 21-chloro-9α-fluoro-16β-methyl-1,4-pregnadiene-11β,17α-diol-3,20-dione 17α-(β-ethoxycarbonyl propionate); 21-chloro-9α-fluoro-16β-methyl-1,4-pregnadiene-11β,17α-diol-3,20-dione 17α-(γ-methoxycarbonyl butyrate); 21-chloro-9α-fluoro-16β-methyl-1,4-pregnadiene-11β,17α-diol-3,20-dione 17α-(γ-ethoxycarbonyl butyrate); 9α,21-difluoro-16β-methyl-1,4-pregnadiene-11β,17α-diol-3,20-dione 17α-(β-methoxycarbonyl propionate); 21-chloro-9α-fluoro-16β-methyl-1,4-pregnadiene-11β,17α-diol-3,20-dione 17α-(β-methoxycarbonyl propionate); 9α,21-dichloro-16β-methyl-1,4-pregnadiene-11β,17α-diol-3,20-dione 17α-(β-methoxycarbonyl propionate); 21-chloro-9α-fluoro-16β-methyl-1,4-pregnadiene-11β,17α-diol-3,6,20-trione 17α-(β-methoxycarbonyl propionate); 21-chloro-9α-fluoro-16α-methyl-1,4-pregnadiene-11β,17α-diol-3,6,20-trione 17α-(β-methoxycarbonyl propionate); 21-chloro-9α-fluoro-16β-methyl-1,4-pregnadiene-11β,17α-diol-3,6,20-trione 17α-(γ-methoxy-

carbonyl butyrate); 21-chloro-9α-fluoro-16β-methyl-1,4-pregnadiene-11β,17α-diol-3,6,20-trione 17α-(β-ethoxycarbonyl propionate); 9α-fluoro-16β-methyl-1,4-pregnadiene-11β,17α,21-triol-3,6,20-trione 17α-(β-methoxycarbonyl propionate) 21-propionate; 9α-fluoro-16β-methyl-1,4-pregnadiene-11β,17α,21-triol-3,6,20-trione 17α-(β-methoxycarbonyl propionate)21-methanesulfonate; 9α-fluoro-16β-methyl-1,4-pregnadiene-11β,17α,21-triol-3,6,20-trione 17α-(β-methoxycarbonyl propionate); 9α-chloro-16β-methyl-1,4-pregnadiene-11β,17α,21-triol-3,6,20-trione 17α-(β-methoxycarbonyl propionate); 6α,9α-difluoro-16β-methyl-1,4-pregnadiene-11β,17α,21-triol-3,20-dione 17α-(β-methoxycarbonyl propionate); 9α-fluoro-6α,16β-dimethyl-1,4-pregnadiene-11β,17α,21-triol-3,20-dione 17α-(β-methoxycarbonyl propionate); 9α-fluoro-16β-methyl-1,4-pregnadiene-6β,11β,17α,21-tetraol-3,20-dione 17α-(β-methoxycarbonyl propionate); 6α,21-difluoro-16β-methyl-1,4-pregnadiene-11β,17α-diol-3,6,20-trione 17α-(β-methoxycarbonyl propionate); etc.

Particularly preferred are 6-oxo 17α-carbonate 21-halogenated derivatives such as 21-chloro-9α-fluoro-16β-methyl-1,4-pregnadiene-11β,17α-diol-3,6,20-trione 17α-methylcarbonate; 21-chloro-9α-fluoro-16β-methyl-1,4-pregnadiene-11β,17α-diol-3,6,20-trione 17α-ethylcarbonate; 21-chloro-9α-fluoro-16β-methyl-1,4-pregnadiene-11β,17α-diol-3,6,20-trione 17α-propylcarbonate; 21-chloro-9α-fluoro-16α-methyl-1,4-pregnadiene-11β,17α-diol-3,6,20-trione 17α-methylcarbonate; 9α,21-difluoro-16β-methyl-1,4-pregnadiene-11β,17α-diol-3,6,20-trione 17α-methylcarbonate, etc., 21-halogenated 17α-alkoxylcarbonyl carboxylate derivatives such as 21-chloro-9α-fluoro-16β-methyl-1,4-pregnadiene-11β,17α-diol-3,20-dione 17α-(β-methoxycarbonyl propionate); 21-chloro-9α-fluoro-16β-methyl-1,4-pregnadiene-11β,17α-diol-3,20-dione 17α-(β-ethoxycarbonyl propionate); 21-chloro-9α-fluoro-16β-methyl-1,4-pregnadiene-11β,17α-diol-3,20-dione 17α-(γ-methoxycarbonyl butyrate); 21-chloro-9α-fluoro-16β-methyl-1,4-pregnadiene-11β,17α-diol-3,20-dione 17α-(γ-ethoxycarbonyl butyrate); 9α,21-difluoro-16β-methyl-1,4-pregnadiene-11β,17α-diol-3,20-dione 17α-(β-methoxycarbonyl propionate); etc. and 6-oxo 21-halogenated 17α-alkoxycarbonyl carboxylate derivatives such as 21-chloro-9α-fluoro-16β-methyl-1,4-pregnadiene-11β,17α-diol-3,6,20-trione 17α-(β-methoxycarbonyl propionate); 21-chloro-9α-fluoro-16β-methyl-1,4-pregnadiene-11β,17α-diol-3,6,20-trione 17α-(β-ethoxycarbonyl propionate); 21-chloro-9α-fluoro 16β-methyl-1,4-pregnadiene-11β,17α-diol-3,6,20-trione 17α-(γ-methoxycarbonyl butyrate); 21-chloro-9α-fluoro-16β-methyl-1,4-pregnadiene-11β,17α-diol-3,6,20-trione 17α-(γ-ethoxycarbonyl butyrate); 9α,21-difluoro-16β-methyl-1,4-pregnadiene-11β,17α-diol-3,6,20-trione 17α-(β-methoxycarbonyl propionate); etc., since they have a very strong topical anti-inflammatory activity.

Now, the preparation of the compounds of the present invention is described according to the definition of $R^1$ of the general formula (I).

(A) The derivatives of the general formula (I) wherein $R^1$ is a hydroxyl group, i.e. 21-ol derivatives represented by the general formula (Ia):

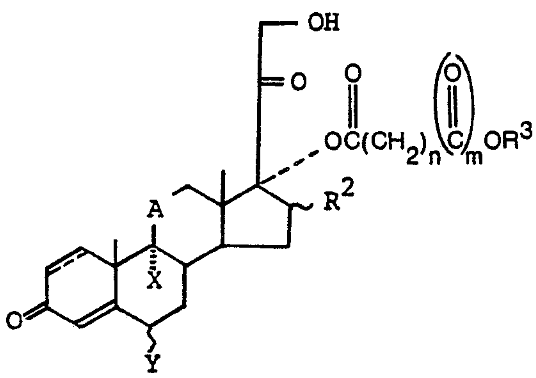

(Ia)

[wherein A, X, Y, $R^2$, m, n and $R^3$ have the same significance as defined in the general formula (I)].

The derivatives are prepared by reacting a 17α,21-diol derivative of the formula (IV) with an orthoester derivative of the formula (V) under acidic conditions to obtain a 17α,21-cyclic orthoester derivative of the formula (VI) and hydrolyzing the 17α,21-cyclic orthoester derivative under acidic conditions. The reaction is shown by the following equation. The 17α,21-diol derivatives of the formula (IV) are described in Arzneim. Forsch., *32(I)*, 317 (1982) and Japanese Unexamined Patent Publication No. 73765/79.

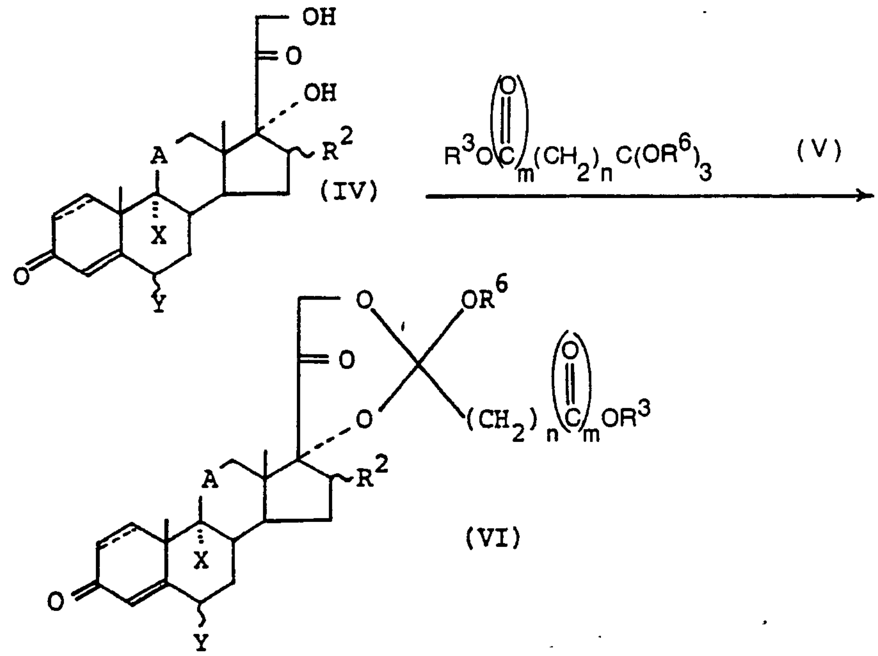

(IV) (V) (VI)

[wherein A, X, Y, $R^2$, $R^3$, m and n have the same significance as defined in the general formula (I) and $R^6$ is an alkyl group having 1 to 10 carbon atoms].

As the orthoester derivatives of the formula (V), for example, methyl orthocarbonate, ethyl orthocarbonate, propyl orthocarbonate, trimethyl ortho β-methoxycarbonyl propionate, triethyl ortho β-ethoxycarbonyl propionate, trimethyl ortho γ-methoxycarbonyl butyrate, triethyl ortho γ-ethoxycarbonyl butyrate, etc. are mentioned and the amount to be used is 1—3 mole per one mole of 17α,21-diol derivatives of the formula (IV).

The reaction to obtain 17α,21-cyclic orthoester derivatives of the formula (VI) is carried out without any solvent or in a solvent such as benzene, dioxane, tetrahydrofuran, methylene chloride, ethyl acetate, etc. As acid catalysts, organic sulfonic acids such as p-toluenesulfonic acid, benzenesulfonic acid, etc., as well as pyridine hydrochloride, sulfuric acid, etc. are used. The reaction temperature is −10—70°C and the reaction period is about 0.5—5 hours.

In the hydrolyzing reaction, organic carboxylic acids such as acetic acid, propionic acid, valeric acid, etc., organic sulfonic acids such as p-toluenesulfonic acid, benzenesulfonic acid, etc. and inorganic acids such as hydrochloric acid, sulfuric acid, etc. are used. However, in this case, 17α-ol derivatives are formed as by-products simultaneously with the desired 21-ol derivatives of the formula (Ia). Therefore, in order to suppress by-production of 17α-ol derivatives, it is preferable to maintain the pH of the reaction mixture at 5 to 6 by adding alkali metal salts of organic acids such as sodium acetate, potassium propionate, etc. in addition to organic carboxylic acids such as acetic acid, propionic acid, etc., or to employ Lewis acids such as aluminum chloride, zinc chloride, etc. but the employment of Lewis acids is more preferable.

Where Lewis acids are employed, aqueous alcohols or aqueous cyclic ethers such as aqueous tetrahydrofuran, aqueous dioxane, etc. are used as a solvent and preferably aqueous alcohols are used. As such alcohols, those represented by the general formula: $R^3OH$ [wherein $R^3$ has the same significance as defined in the general formula (I)] are desired. Use of other alcohols is undesirable since it is probable to induce transesterification reaction resulting in the contamination of the reaction product. Content of water in the alcohol is normally 5 to 40 weight %. The reaction temperature is 0 to 50°C and the reaction period is 0.5 to 4 hours.

(B) The derivatives of the general formula (I) wherein $R^1$ is a group $—OSO_2R^4$, i.e. 21 sulfonate derivatives represented by the general formula (Ib):

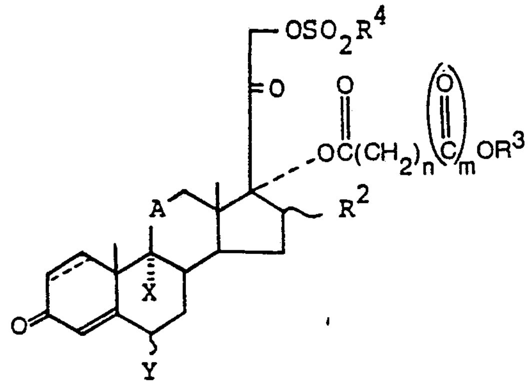

(Ib)

[wherein A, X, Y, $R^2$, m, n, $R^3$ and $R^4$ have the same significance as defined in the general formulae (I) and (II)]. The derivatives are prepared by reacting 21-ol derivatives of the formula (Ia) obtained in above (A) with sulfonic acid anhydride or sulfonic acid halide represented by the general formula (IIa) or (IIb):

$$(R^4SO_2)_2O \qquad \text{(IIa)}$$

$$R^4SO_2X^1 \qquad \text{(IIb)}$$

[wherein $R^4$ has the same significance as defined in the general formula (II) and $X^1$ is a halogen atom].

Such sulfonic acid derivatives include, for example, methanesulfonyl chloride, p-toluenesulfonyl chloride, trifluoromethanesulfonyl chloride, trifluoromethanesulfonic acid anhydride, etc. The amount to be used is 1 to 3 mols per one mol of 21-ol derivatives of the formula (Ia).

As the solvent, aromatic amines such as pyridine and aliphatic tertiary amines such as triethyl amine are used, which may be diluted with halogenated hydrocarbons such as methylene chloride, dichloroethane, etc.

The reaction temperature is from a room temperature to −40°C and the reaction period is from 5 minutes to 2 hours.

(C) The derivatives of the general formula (I) wherein $R^1$ is a group —$OCOR^5$, i.e. 21-carboxylate derivatives represented by the general formula (Ic):

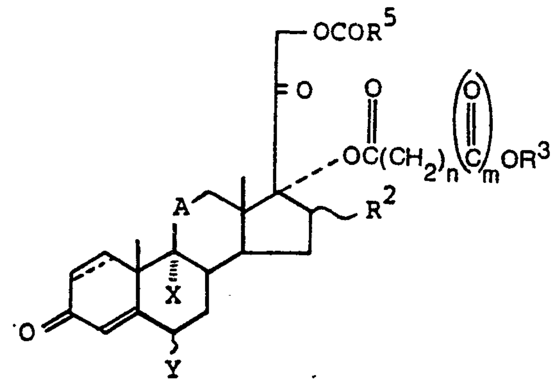

(Ic)

[wherein A, X, Y, m, n, $R^2$, $R^3$ and $R^5$ have the same significance as defined in the general formulae (I) and (III)]. These derivatives are prepared by reacting 21-ol derivatives of the formula (Ia) obtained in (A) above with carboxylic acid anhydride or carboxylic acid halide represented by the general formula (IIIa) or (IIIb):

$$(R^5CO)_2O \qquad \text{(IIIa)}$$

$$R^5COX^1 \qquad \text{(IIIb)}$$

[wherein $R^5$ has the same significance as defined in the general formula (III) and $X^1$ is a halogen atom].

As the carboxylic acid derivatives, for example, acetic acid anhydride, propionic acid anhydride, butyryl chloride and cyclopropylcarbonyl chloride are mentioned. The amount to be used is 1 to 3 mols per 1 mol of 21-ol derivatives of the general formula (Ia).

As the solvent, aromatic amines such as pyridine, aliphatic tertiary amines such as triethyl amine are used, which may be diluted with halogenated hydrocarbons such as methylene chloride, dichloroethane, etc.

The reaction temperature is from −30°C to 50°C and the reaction period is 5 minutes to 3 hours.

(D) The derivatives of the general formula (I) wherein $R^1$ is a halogen atom, i.e. 21-halogenated corticoids represented by the general formula (Id):

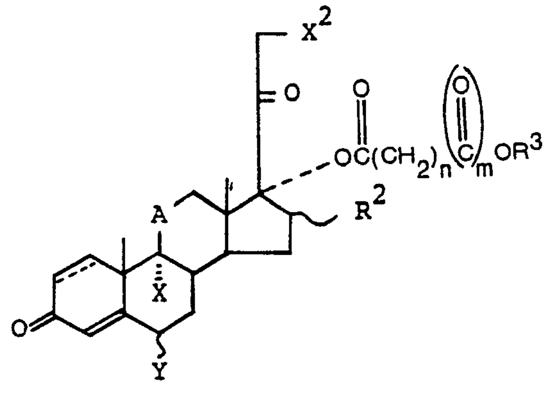

(Id)

[wherein A, X, Y, m, n, $R^2$ and $R^3$ have the same significance as defined in the general formula (I) and $X^2$ is a halogen atom]. The compounds are prepared by reacting 21-sulfonate derivatives of the formula (Ib) obtained according to the process described in (B) above with a halogen ion donor.

As the halogen ion donor, lithium chloride, lithium bromide, lithium iodide, potassium chloride, etc. are mentioned. The amount of the halogen ion donor to be used is 1 to 10 mols per 1 mol of 21-sulfonate derivatives of the formula (Ia).

As examples of 21-sulfonate, methane sulfonate, p-toluene sulfonate and trifluoromethane sulfonate are mentioned.

The reaction is carried out in an aprotic solvent such as dimethylformamide, dimethylacetamide, dimethylsulfoxide, etc. at 0—120°C. Usually, the reaction period is 0.5—20 hours.

Further, 21-halogenated corticoids represented by the general formula (Id)':

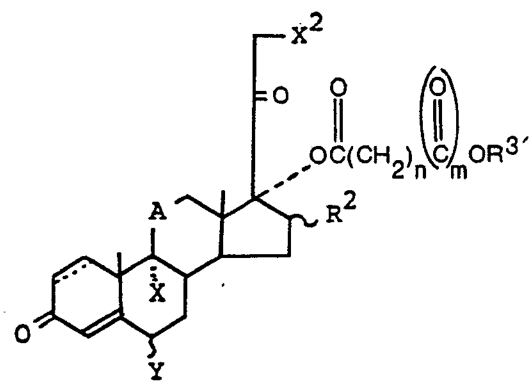

(Id)'

[wherein A, X, Y, m, n, $X^2$ and $R^2$, have the same significance as defined in the general formulae (Id) and $R^{3'}$ is an alkyl group having 1 to 10 carbon atoms and is different from $R^3$ of the general formula (Id)] are obtained by reacting 21-halogenated corticoids of the formula (Id) with alcohols represented by the general formula: $R^{3'}OH$ (VII) [wherein $R^{3'}$ has the same significance as defined in the general formula (Id)'] to effect transesterification.

In the above transesterification reaction, inorganic acids such as hydrogen chloride, sulfuric acid, etc., alkali metal hydroxides, such as sodium hydroxide and potassium hydroxide and alkali metal compounds of alcohols which correspond to the desired ester, such as sodium methoxide, sodium ethoxide, sodium propoxide, potassium methoxide, etc. are used as catalysts. As the solvent, an excess amount of nonaqueous alcohols such as methanol, ethanol, propanol, etc. which correspond to the desired ester is used.

The reaction temperature is 0—60°C and the reaction period is 1 to 20 hours.

The corticoid derivatives of the formula (I) obtained in the above (A)—(D) can be purified by recrystallization.

The corticoid derivatives (I) of the present invention have a strong topical anti-inflammatory activity and are weak in systemic side effects. Therefore, they are very useful as anti-inflammatory agents, particularly, as topical anti-inflammatory agents.

The derivatives of the present invention may be formulated into a preparation suitable for topical

administration in conventional manner with the aid of one or more carriers or excipients. Examples of types of preparation include ointments, lotions, creams, sprays, powders, drops (e.g., ear drops and eye drops), suppositories or retention enemas (e.g., for the treatment of rectal or colonic inflammations) and tablets or pellets (e.g., for the treatment of aphthous ulcers) and aerosols.

The proportion of active steroid in the compositions according to the invention depends on the precise type of formulations to be prepared but will generally be within the range of from 0.0001% to 5% by weight. Generally, however, for most types of preparations advantageously the proportion used will be within the range of from 0.001 to 0.5% and preferably 0.01 to 0.25%.

Now, the present invention is explained more in detail referring to examples but it can not be limited to the examples so far as it is within the gist thereof.

Reference Example

Example of the Preparation of the Starting Compound in Examples 1, 3 and 5)

Synthesis of 9α-fluoro-16β-methyl-1,4-pregnadiene-11β,17α,21-triol-3,6,20-trione 17α,21-dimethyl orthocarbonate

To 1.0 g of 9α-fluoro-16β-methyl-1,4-pregnadiene-11β,17α,21-triol-3,6,20-trione are added 20 ml of tetrahydrofuran, 1.0 ml of methyl orthocarbonate and 0.04 g of p-toluenesulfonic acid, and the mixture is stirred at room temperature for 3 hours.

The resulting reaction mixtures is poured into ice water containing sodium bicarbonate and extraction is carried out with ethyl acetate. The organic layer is washed with water and dried. After the solvent is removed by distillation, about 1 g of yellow crystals are obtained.

The yellow crystals are dissolved in 5 ml of chloroform and passed through a column packed with 40 g of silica gel. Elution is then carried out with chloroform-ethyl acetate (2:1) and the solvent is removed from the eluate by distillation. As the result, 1.22 g of 9α-fluoro-16β-methyl-1,4-pregnadiene-11β,17α,21-triol-3,6,20-trione 17α,21-dimethyl orthocarbonate are obtained. Analysis by thin layer chromatography [silica gel 0.25 mm, chloroform-ethyl acetate (2:1)] reveals one spot. The product is used as a starting material for the next step without further purification.

When ethyl orthocarbonate and propyl orthocarbonate are reacted respectively in the same manner in place of methyl orthocarbonate, 9α-fluoro-16β-methyl-1,4-pregnadiene-11β,17α,21-triol-3,6,20-trione 17α,21-diethyl orthocarbonate and 9α-fluoro-16β-methyl-1,4-pregnadiene-11β,17α,21-triol-3,6,20-trione 17α,21-dipropyl orthocarbonate are obtained.

Example 1

9a-fluoro-16β-methyl-1,4-pregnadiene-11β,17α,21-triol-3,6,20-trione 17α-methyl carbonate

1.0 g (2.09 mmols) of 9α-fluoro-16β-methyl-1,4-pregnadiene-11β,17α,21-triol-3,6,20-trione 17α,21-dimethyl orthocarbonate is added to a solution mixture of 123 ml of methanol and 50 ml of 0.56% aqueous solution of aluminum chloride. The mixture is stirred at 40°C for one hour. The resulting reaction mixture is poured into 600 ml of saturated saline solution. Extraction is carried out with ethyl acetate and the solvent is removed by distillation. As the result, 1.13 g of an oily matter is obtained.

The oily matter is charged in a column packed with 40 g of silica gel and eluted with 2% methanol-dichloroethane. After the solvent is removed with the eluate by distillation, 0.71 g of 9α-fluoro-16β-methyl-1,4-pregnadiene-11β,17α,21-triol-3,6,20-trione 17α-methylcarbonate are obtained.

Melting point: 160—162°C.

Example 2

21-chloro-9a-fluoro-16β-methyl-1,4-pregnadiene-11β,17α-diol-3,6,20-trione 17α-methyl carbonate

0.75 ml of pyridine and 20 ml of methylene chloride are added to 0.54 g (1.16 mmols) of 9α-fluoro-16β-methyl-1,4-pregnadiene-11β,17α,21-triol-3,6,20-trione 17α-methylcarbonate, obtained in Example 1 and the mixture is cooled at −20°C.

0.35 ml (2.08 mmols) of trifluoromethane sulfonic acid anhydride are added thereto and the mixture is stirred for 30 minutes. The resulting reaction mixture is poured into 50 ml of ice water containing 1 ml of hydrochloric acid and the hydrochloric acid layer is separated, which is washed successively with 5% aqueous solution of hydrochloric acid, 5% aqueous solution of sodium bicarbonate and saturated saline solution and dried.

The thus obtained methylene chloride solution is concentrated and 3.5 g of a solution of 9α-fluoro-16β-methyl-1,4-pregnadiene-11β,17α,21-triol-3,6,20-trione 17α-methylcarbonate 21-trifluoromethane sulfonate in methylene chloride are obtained.

To the solution are added 7.5 ml of dimethylformamide and 0.5 g of lithium chloride, and the mixture is stirred at room temperature for 40 minutes. The reaction solution is poured into 50 ml of ice water, and extraction is carried out with 50 ml of methylene chloride. The extract is concentrated to give 0.62 g of an oily matter.

The oily matter is charged in a column packed with 25 g of silica gel and eluted with benzene-ethylacetate (2:1). After the solvent is removed by distillation, 0.54 g of 21-chloro-9α-fluoro-16β-methyl-1,4-pregnadiene-11β,17α-diol-3,6,20-trione 17α-methyl carbonate are obtained. The product is recrystallized from ethyl acetate-n-hexane.

Melting point: 185—186°C.
Elementary analysis:

| | | |
|---|---|---|
| Found (%): | C, 59.35; | H, 5.98 |
| Calcd. (%) for $C_{24}H_{28}O_7ClF$: | C, 59.69; | H, 5.84 |

Example 3

9α-fluoro-16β-methyl-1,4-pregnadiene-11β,17α,21-triol-3,6,20-trione 17α-ethyl carbonate

1.1 g of 9α-fluoro-16β-methyl-1,4-pregnadiene-11β,17α,21-triol-3,6,20-trione 17α,21-diethyl orthocarbonate are added to a mixture of 123 ml of ethanol and 50 ml of 0.56% aqueous solution of aluminum chloride, and the mixture is stirred at 40°C for one hour. The reaction mixture is treated in the same manner as described in Example 1. After carrying out column chromatography, 0.79 g of 9α-fluoro-16β-methyl-1,4-pregnadiene-11β,17α,21-triol-3,6,20-trione 17α-ethyl carbonate are obtained.

Melting point: 130—133°C.

Example 4

21-chloro-9a-fluoro-16β-methyl-1,4-pregnadiene-11β,17α-diol-3,6,20-trione 17α-ethyl carbonate

To 0.60 g of 9α-fluoro-16β-methyl-1,4-pregnadiene-11β,17α,21-triol-3,6,20-trione 17α-ethyl carbonate obtained in Example 3 are added 0.75 ml of pyridine and 20 ml of methylene chloride, and the mixture is cooled to −20°C. 0.35 ml of trifluoromethane sulfonic acid anhydride are added thereto and stirred for 30 minutes. The reaction mixture is treated in the same manner as described in Example 2 and 4 g of a solution of 9α-fluoro-16β-methyl-1,4-pregnadiene-11β,17α,21-triol-3,6,20-trione 17α-ethyl carbonate 21-trifluoromethane sulfonate in methylene chloride are obtained.

To the solution, 7.5 ml of dimethylformamide and 0.5 g of lithium chloride are added and stirred at room temperature for one hour. Thereafter, the procedure described in Example 2 is followed. After carrying out column chromatography, 0.6 g of 21-chloro-9α-fluoro-16β-methyl-1,4-pregnadiene-11β,17α-diol-3,6,20-trione 17α-ethyl carbonate are obtained. The product is recrystallized from benzene-n-hexane.

Melting point: 202°C (decomposition).
Elementary analysis:

| | | |
|---|---|---|
| Found (%): | C, 60.65; | H, 6.12 |
| Calcd. (%) for $C_{25}H_{30}O_7ClF$: | C, 60.42; | H, 6.08 |

Example 5

9a-fluoro-16β-methyl-1,4-pregnadiene-11β,17α,21-triol-3,6,20-trione 17α-propyl carbonate

1.67 g of 9α-fluoro-16β-methyl-1,4-pregnadiene-11β,17α,21-triol-3,6,20-trione 17α,21-dipropyl orthocarbonate are added to 187 ml of n-propanol and 80 ml of 0.56% aqueous solution of aluminum chloride, and the mixture is stirred at 40°C for one hour. The reaction mixture is treated in the same manner as described in Example 1. After carrying out column chromatography, 0.86 g of 9α-fluoro-16β-methyl-1,4-pregnadiene-11β,17α,21-triol-3,6,20-trione 17α-propyl carbonate are obtained.

Melting point: 197—200°C (decomposition).

Example 6

21-chloro-9a-fluoro-16β-methyl-1,4-pregnadiene-11β,17α-diol-3,6,20-trione 17α-propyl carbonate

To 0.70 g of 9α-fluoro-16β-methyl-1,4-pregnadiene-11β,17α,21-triol-3,6,20-trione 17α-propyl carbonate obtained in Example 5 are added 0.75 ml of pyridine and 20 ml of methylene chloride, and the mixture is cooled to −20°C. 0.35 ml of trifluoromethane sulfonic acid anhydride are added thereto and the mixture is stirred for 25 minutes. The reaction mixture is treated in the same manner as described in Example 2 and 2.7 g of a solution of 9α-fluoro-16β-methyl-1,4-pregnadiene-11β,17α,21-triol-3,6,20-trione 17α-propyl carbonate 21-trifluoromethane sulfonate in methylene chloride is obtained.

7.5 ml of dimethylformamide and 0.5 g of lithium chloride are added to the solution and the mixture is stirred at room temperature for one hour. Thereafter, the procedure described in Example 2 is followed. After carrying out column chromatography, 0.45 g of 21-chloro-9α-fluoro-16β-methyl-1,4-pregnadiene-11β,17α-diol-3,6,20-trione 17α-propyl carbonate are obtained. The product is recrystallized from ethyl acetate-n-hexane.

Melting point: 200—202°C (decomposition).

Example 7

9a-fluoro-16α-methyl-1,4-pregnadiene-11β,17α,21-triol-3,6,20-trione 17α-methyl carbonate

1.3 g of 9α-fluoro-16α-methyl-1,4-pregnadiene-11β,17α,21-triol-3,6,20-trione 17α,21-dimethyl orthocarbonate are added to a mixed solution of 30 ml of methanol, 21 ml of tetrahydrofuran and 5 ml of 0.56% aqueous solution of aluminum chloride, and the mixture is stirred at 40°C for 1.5 hours. The reaction mixture is treated in the same manner as described in Example 1. After carrying out column chromatography, 0.97 g of 9α-fluoro-16α-methyl-1,4-pregnadiene-11β,17α,21-triol-3,6,20-trione 17α-methyl carbonate are obtained.

Melting point: 248—251°C (decomposition).

Example 8

21-chloro-9a-fluoro-16α-methyl-1,4-pregnadiene-11β,17α-diol-3,6,20-trione 17α-methyl carbonate

To 0.6 g (1.29 mmols) of 9α-fluoro-16α-methyl-1,4-pregnadiene-11β,17α,21-triol-3,6,20-trione 17α-methyl carbonate obtained in Example 7 are added 0.73 ml of pyridine and 20 ml of methylene chloride, and the mixture is cooled to −20°C. 0.34 ml (2.02 mmols) of trifluoromethane sulfonic acid anhydride are added thereto and the mixture is stirred for 30 minutes. The reaction mixture is treated in the same manner as described in Example 2 and 3 g of a solution of 9α-fluoro-16α-methyl-1,4-pregnadiene-11β,17α,21-triol-3,6,20-trione 17α-methyl carbonate 21-trifluoromethane sulfonate in methylene chloride are obtained.

7.5 ml of dimethylformamide and 0.5 g of lithium chloride are added to the solution, and the mixture is stirred at room temperature for one hour. Thereafter, the procedure described in Example 2 is followed. After carrying out column chromatography, 0.41 g of 21-chloro-9α-fluoro-16α-methyl-1,4-pregnadiene-11β,17α-diol-3,6,20-trione 17α-methyl carbonate are obtained. The product is recrystallized from ethyl acetate-n-hexane.

Melting point: 268—271°C (decomposition).

Elementary analysis:

| | | |
|---|---|---|
| Found (%): | C, 59.64; | H, 5.96 |
| Calcd. (%) for $C_{24}H_{28}O_7ClF$: | C, 59.68; | H, 5.85 |

Comparative Example 1

21-chloro-9a-fluoro-16β-methyl-1,4-pregnadiene-11β,17α-diol-3,6,20-trione 17α-propionate

To 0.285 g of 9α-fluoro-16β-methyl-1,4-pregnadiene-11β,17α,21-triol-3,6,20-trione 17α-propionate are added 3 ml of pyridine and 0.06 ml of methane sulfonylchloride and the mixture is stirred at room temperature for 30 minutes.

The resulting reaction mixture is subjected to extraction with methylene chloride and extract is successively washed with 5% HCl, aqueous sodium bicarbonate solution and aqueous saline solution. After drying, methylene chloride is removed by distillation to obtain 9α-fluoro-16β-methyl-1,4-pregnadiene-11β,17α,21-triol-3,6,20-trione 17α-propionate 21-methane sulfonate.

3 ml of dimethylformamide and 0.3 g of lithium chloride are added thereto and the mixture is stirred at 80°C for 8 hours. The reaction mixture is poured to a large volume of water and the crystals formed are separated by filtration. After drying, the crystals are dissolved in 2 ml of benzene, passed through a column packed with silica gel and eluted with ethyl acetate-benzene (5:95). After removing the solvent by distillation, 0.220 g of 21-chloro-9α-fluoro-16β-methyl-1,4-pregnadiene-11β,17α-diol-3,6,20-trione 17α-propionate are obtained. The product is crystallized from ethyl acetate.

Melting point: 228.5°C (decomposition).

Comparative Example 2

21-chloro-9α-fluoro-16β-methyl-1,4-pregnadiene-11β,17α-diol-3,20-dione 17α-methyl carbonate

To 0.50 g of 9α-fluoro-16β-methyl-1,4-pregnadiene-11β,17α,21-triol-3,20-dione 17α-methyl carbonate are added 20 ml of methylene chloride, 0.75 ml of pyridine and 0.35 ml of trifluoromethane sulfonic acid anhydride and the mixture is stirred at −20°C for 1.5 hours. The reaction mixture is treated in the same manner as described in Example 2 and 5 ml of a solution of 9α-fluoro-16β-methyl-1,4-pregnadiene-11β,17α,21-triol-3,20-dione 17α-methyl carbonate 21-trifluoromethane sulfonate in methylene chloride are obtained.

7.5 ml of dimethylformamide and 0.5 g of lithium chloride are added to the solution and the mixture is stirred at room temperature for 1.5 hours. Thereafter, the procedure described in Example 2 is followed. After carrying out column chromatography, 0.532 g of 21-chloro-9α-fluoro-16β-methyl-1,4-pregnadiene-11β,17α-diol-3,20-dione 17α-methyl carbonate are obtained. The product is recrystallized from ethyl acetate.

Melting point: 196—198°C (decomposition).

Example 9

4-pregnene-11β,17α,21-triol-3,20-dione 17α-(β-methoxycarbonyl propionate)

To 1.0 g of 4-pregnene-11β,17α,21-triol-3,20-dione are added 15 ml of tetrahydrofuran, 0.02 g of p-toluenesulfonic acid and 1.33 g of trimethyl ortho β-methoxycarbonyl propionate and the mixture is stirred at room temperature for 3 hours.

The resulting reaction mixture is poured into ice water containing sodium bicarbonate and extraction is carried out with ethyl acetate. The organic layer is washed with water and dried with magnesium sulfate. After removing the solvent by distillation, 2.2 g of an oily matter is obtained.

The oily matter is dissolved in chloroform and charged in a column packed with 40 g of silica gel. Elution is carried out with benzene-ethyl acetate (4:1) and the solvent is removed by distillation. After purification, 0.52 g of 4-pregnene-11β,17α,21-triol-3,20-dione 17α,21-methyl ortho β-methoxycarbonyl propionate is obtained as an amorphous solid.

0.52 g of 4-pregnene-11β,17α,21-triol-3,20-dione 17α,21-methyl ortho β-methoxycarbonyl propionate are dissolved into 20 ml of methanol. To the solution is added 0.8 ml of 0.28% aqueous solution of aluminum chloride and the mixture is stirred at room temperature for 6 hours. The reaction mixture is

poured into 100 ml of saturated saline solution. Extraction is carried out with ethyl acetate followed by washing with water and drying. After the solvent is removed by distillation, the resulting residue is subjected to purification by chromatography using silica gel (benzene:ethyl acetate = 3:1). As the result, 0.43 g of 4-pregnene-11β,17α,21-triol-3,20-dione 17α-(β-methoxycarbonyl propionate) is obtained as an amorphous solid.

IR: 3460, 2950, 1735, 1660 $cm^{-1}$.

NMR ($CDCl_3$, δ): 0.93 (S, 18-$CH_3$), 1.43 (S, 19-$CH_3$), 2.60 (S, 17-$OCOCH_2CH_2COO$—), 4.28 (d, 21-$CH_2$), 5.70 (S, 4-CH).

## Example 10

9α-fluoro-16β-methyl-1,4-pregnadiene-11β,17α,21-triol-3,20-dione 17α-(β-methoxycarbonyl propionate)

To 2.0 g of 9α-fluoro-16β-methyl-1,4-pregnadiene-11β,17α,21-triol-3,20-dione are added 21 ml of tetrahydrofuran, 2.5 g of trimethyl ortho β-methoxycarbonyl propionate and 0.03 g of p-toluene sulfonic acid and the mixture is stirred at room temperature for 3 hours and then left standing overnight.

The resulting reaction mixture is poured into ice water containing sodium bicarbonate and extraction is carried out with ethyl acetate. After washing with water and drying, the solvent is removed by distillation to give 4.0 g of a residue.

The residue is subjected to purification by column chromatography using silica gel (benzene:ethyl acetate = 4:1) and 2.0 g of 9α-fluoro-16β-methyl-1,4-pregnadiene-11β,17α,21-triol-3,20-dione 17α,21-methyl ortho β-methoxycarbonyl propionate is obtained as an amorphous solid.

2.0 g of 9α-fluoro-16β-methyl-1,4-pregnadiene-11β,17α,21-triol-3,20-dione 17α,21-methyl ortho β-methoxycarbonyl propionate is dissolved in 55 ml of methanol and 3.3 ml of 0.28% aqueous solution of aluminum chloride is added thereto. Reaction is carried out at room temperature for 5 hours and the reaction mixture is left standing overnight. The reaction mixture is then poured into 300 ml of saturated saline solution and extraction is carried out with ethyl acetate. After washing with water and drying, the solvent is removed by distillation to give 1.88 g of crystals. The crystals are recrystallized from ethyl acetate-n-hexane. As the result, 1.78 g of 9α-fluoro-16β-methyl-1,4-pregnadiene-11β,17α,21-triol-3,20-dione 17α-(β-methoxycarbonyl propionate) are obtained.

Melting point: 227—230°C.

IR: 3460, 2950, 1735, 1665 $cm^{-1}$.

NMR (DMSO-$d_6$, δ): 0.88 (S, 18-$CH_3$), 1.48 (S, 19-$CH_3$), 2.57 (S, 17-$OCOCH_2CH_2COO$—), 3.40 (S, 17-$CO_2CH_3$), 5.33 (S, 11β-OH), 6.0 (S, 4-CH), 6.2 (d-d, 2-CH), 7.27 (d, 1-CH).

## Example 11

9α-fluoro-16β-methyl-1,4-pregnadiene-11β,17α,21-triol-3,6,20-trione 17α-(β-methoxycarbonyl propionate)

To 6.51 g of 9α-fluoro-16β-methyl-1,4-pregnadiene-11β,17α,21-triol-3,6,20-trione are added 61 ml of tetrahydrofuran, 9.23 g of trimethyl ortho β-methoxycarbonyl propionate and 0.11 g of p-toluene sulfonic acid. Reaction is carried out at room temperature for 5 hours and then the reaction mixture is left standing overnight.

The reaction mixture is poured into ice water containing sodium bicarbonate and extraction is carried out with ethyl acetate. After washing with water and drying, the solvent is removed by distillation to give 15.7 g of a residue.

After purification by chromatography using silica gel (benzene:ethyl acetate = 4:1), 5.79 g of crystals of 9α-fluoro-16β-methyl-1,4-pregnadiene-11β,17α,21-triol-3,6,20-trione 17α,21-methyl ortho β-methoxycarbonyl propionate are obtained (Melting point: 180—185°C).

5.70 g of 9α-fluoro-16β-methyl-1,4-pregnadiene-11β,17α,21-triol-3,6,20-trione 17α,21-methyl ortho β-methoxycarbonyl propionate are dissolved in 180 ml of methanol and 60 ml of ethyl acetate and 8.6 ml of 0.28% aqueous solution of aluminum chloride is added thereto. Reaction is carried out at room temperature for 5 hours and the reaction mixture is left standing overnight. The reaction mixture is then poured into 600 ml of saturated saline solution and extraction is carried out with ethyl acetate. After washing with water and drying, the solvent is removed by distillation. As the result, 5.41 g of 9α-fluoro-16β-methyl-1,4-pregnadiene-11β,17α,21-triol-3,6,20-trione 17α-(β-methoxycarbonyl propionate) are obtained as crystals.

Melting point: 175—179°C.

IR: 3460, 2950, 1735, 1705, 1663 $cm^{-1}$.

NMR ($CDCl_3$, δ): 0.98 (S, 18-$CH_3$), 1.50 (S, 19-$CH_3$), 2.62 (S, 17-$OCOCH_2CH_2COO$—), 6.35 (S, 4-CH).

## Example 12

21-chloro-9a-fluoro-16β-methyl-1,4-pregnadiene-11β,17α-diol-3,20-dione 17α-(β-methoxycarbonyl propionate)

To 690 mg of 9α-fluoro-16β-methyl-1,4-pregnadiene-11β,17α,21-triol-3,20-dione 17α-(β-methoxycarbonyl propionate) obtained in Example 10 are added 10 ml of methylene chloride and 0.51 ml of triethylamine and further 0.51 ml of methanesulfonyl chloride is added thereto under ice cooling. After 10 minutes, the reaction mixture is restored to room temperature, and stirred for 30 minutes. Thereafter, 30 ml of methylene chloride is added to the reaction mixture, which is washed successively with 1N-hydrochloric acid, saturated aqueous solution of sodium hydrogen carbonate and saturated saline solution and dried.

After removing the solvent by distillation, 729 mg of 9α-fluoro-16β-methyl-1,4-pregnadiene-11β,17α,21-triol-3,20-dione 17α-(β-methoxycarbonyl propionate)21-methanesulfonate is obtained as an amorpous solid.

To 729 mg of 9α-fluoro-16β-methyl-1,4-pregnadiene-11β,17α,21-triol-3,20-dione 17α-(β-methoxycarbonyl propionate)21-methanesulfonate are added 8 ml of dimethylformamide and 404 mg of lithium chloride and the mixture is stirred at 80°C for 11 hours.

Dimethylformamide is removed by distillation under reduced pressure and methylene chloride is added to the residue. The mixture is thoroughly washed with water and dried. After the solvent is removed by filtration, 651 mg of an oily matter is obtained. Purification is carried out by column chromatography using silica gel and 547 mg of 21-chloro-9α-fluoro-16β-methyl-1,4-pregnadiene-11β,17α-diol-3,20-dione 17α-(β-methoxycarbonyl propionate) are obtained as crystals. Recrystallization is carried out from ethyl acetate-n-hexane.

Melting point: 215—216°C.

Elementary analysis:

| | | |
|---|---|---|
| Found (%): | C, 61.98; | H, 6.50 |
| Calcd. (%) for $C_{27}H_{34}O_7ClF$: | C, 61.77; | H, 6.53 |

IR: 3460, 3000, 1745, 1680 $cm^{-1}$.

NMR (DMSO-$d_6$, δ): 0.88 (S, 18-$CH_3$), 1.50 (S, 19-$CH_3$), 2.61 (S, 17-$OCOCH_2CH_2COO$—), 3.55 (S, 17-$COOCH_3$), 4.71 (S, 21-$CH_2$), 6.0 (S, 4-CH), 6.22 (d-d, 2-CH), 7.27 (d, 1-CH).

## Example 13

21-chloro-9α-fluoro-16β-methyl-1,4-pregnadiene-11β,17α-diol-3,6,20-trione 17α-(β-methoxycarbonyl propionate)

To 5.31 g of 9α-fluoro-16β-methyl-1,4-pregnadiene-11β,17α,21-triol-3,6,20-trione 17α-(β-methoxycarbonyl propionate) obtained in Example 11 are added 105 ml of methylene chloride and 3.86 mg of triethylamine and 1.03 ml of methanesulfonyl chloride is further added thereto under ice cooling. After ten minutes, the reaction mixture is restored to room temperature and stirred for 50 minutes. Then, the reaction mixture is washed with 1N-hydrochloric acid, saturated aqueous solution of sodium hydrogen carbonate and saturated saline solution and dried.

After removing the solvent by distillation, 6.31 g of 9α-fluoro-16β-methyl-1,4-pregnadiene-11β,17α,21-triol-3,6,20-trione 17α-(β-methoxycarbonyl propionate)21-methane sulfonate is obtained as an amorphous solid.

To 6.31 g of 9α-fluoro-16β-methyl-1,4-pregnadiene-11β,17α,21-triol-3,6,20-trione 17α-(β-methoxycarbonyl propionate) 21-methane sulfonate are added 45 ml of dimethylformamide and 3.03 g of lithium chloride and the mixture is stirred at 80°C for 15 hours.

Dimethylformamide is removed by distillation under reduced pressure and methylene chloride is added to the residue. After thoroughly washing with water and drying, the solvent is removed by distillation to give 5.7 g of a residue.

Purification is carried out by chromatography using silica gel (benzene:ethyl acetate = 4:1) and 3.83 g of 21-chloro-9α-fluoro-16β-methyl-1,4-pregnadiene-11β,17α-diol-3,6,20-trione 17α-(β-methoxycarbonyl propionate) are obtained as crystals. Recrystallization is carried out from ethyl acetate-n-hexane.

Melting point: 183—187°C.

IR: 3460, 2950, 1735, 1705, 1660 $cm^{-1}$.

NMR ($CDCl_3$, δ): 1.03 (S, 18-$CH_3$), 1.57 (S, 19-$CH_3$), 2.65 (S, 17-$OCOCH_3$), 4.07 (S, 21-$CH_2$), 6.50 (S, 4-CH).

## Example 14

21-chloro-9α-fluoro-16α-methyl-1,4-pregnadiene-11β,17α-diol-3,20-dione 17α-(β-methoxycarbonyl propionate)

To 2.0 g of 9α-fluoro-16α-methyl-1,4-pregnadiene-11β,17α,21-triol-3,20-dione are added 20 ml of tetrahydrofuran, 2.45 g of trimethyl ortho β-methoxycarbonyl propionate and 0.03 g of p-toluene sulfonic acid. Reaction is carried out at room temperature for 5 hours and the reaction mixture is left standing overnight.

The reaction mixture is poured to ice water containing sodium bicarbonate and extraction is carried out with ethyl acetate. After washing with water and drying, the solvent is removed by distillation. The resulting residue is purified by chromatography using silica gel (benzene:ethyl acetate = 4:1) and 1.71 g of 9α-fluoro-16α-methyl-1,4-pregnadiene-11β,17α,21-triol-3,20-dione 17α,21-methyl ortho β-methoxycarbonyl propionate are obtained as crystals.

To 1.71 g of 9α-fluoro-16α-methyl-1,4-pregnadiene-11β,17α,21-triol-3,20-dione 17α,21-methyl orthomethoxycarbonyl propionate are added 80 ml of methanol, 20 ml of ethyl acetate and 4 ml of 0.28% aqueous solution of aluminum chloride and the mixture is stirred at 40°C for 2 hours. The reaction mixture is poured into saturated saline solution and extraction is carried out with ethyl acetate. After washing with water and drying, the solvent is removed by distillation to give 1.70 g of crude crystals of 9α-fluoro-16α-methyl-1,4-pregnadiene-11β,17α,21-triol-3,20-dione 17α-(β-methoxycarbonyl propionate) is obtained.

To 1.70 g of 9α-fluoro-16α-methyl-1,4-pregnadiene-11β,17α,21-triol-3,20-dione 17α-(β-methoxycarbonyl propionate) are added 25 ml of methylene chloride and 1.27 ml of triethylamine and 0.34 ml of methanesulfonyl chloride is further added thereto under ice-cooling. After 10 minutes, the mixture is

restored to room temperature and stirred for 30 minutes.

25 ml of methylene chloride is added to the reaction mixture and the resulting mixture is washed with 1N-hydrochloric acid, saturated aqueous solution of sodium hydrogen carbonate and saturated saline solution and then dried. After removing the solvent by distillation, 1.89 g of 9α-fluoro-16α-methyl-1,4-pregnadiene-11β,17α,21-triol-3,20-dione 17α-(β-methoxycarbonyl propionate)21-methanesulfonate is obtained as an amorphous solid.

To 1.89 g of 9α-fluoro-16α-methyl-1,4-pregnadiene-11β,17α,21-triol-3,20-dione 17α-(β-methoxycarbonyl propionate)21-methanesulfonate are added 1.0 g of lithium chloride and 30 ml of dimethylformamide and the mixture is stirred at 80°C for 20 hours.

The solvent is removed by distillation under reduced pressure and methylene chloride is added thereto. The mixture is thoroughly washed with water and dried. Then, methylene chloride is removed by distillation and the residue is purified by chromatography using silica gel (benzene:ethyl acetate = 4:1). As the result, 1.11 g of crystals of 21-chloro-9α-fluoro-16α-methyl-1,4-pregnadiene-11β,17α-diol-3,20-dione 17α-(β-methoxycarbonyl propionate) are obtained.

Melting point: 210—214°C.

IR: 3460, 2960, 1740, 1670 $cm^{-1}$.

NMR ($CDCl_3$, δ): 1.07 (S, 18-$CH_3$), 1.55 (S, 19-$CH_3$), 2.62 (S, 17-$OCOCH_2CH_2COO$—), 3.65 (S, 17-$COOCH_3$), 4.03 (S, 21-$CH_2$), 6.10 (S, 4-CH), 6.27 (d-d, 2-CH).

## Example 15

21-chloro-9α-fluoro-16β-methyl-1,4-pregnadiene-11β,17α-diol-3,20-dione 17α-(γ-ethoxycarbonyl butyrate)

To 2.0 g of 9α-fluoro-16β-methyl-1,4-pregnadiene-11β,17α,21-triol-3,20-dione are added 35 ml of tetrahydrofuran, 2.0 g of triethyl ortho γ-ethoxycarbonyl butyrate and 40 mg of p-toluenesulfonic acid and the mixture is stirred at room temperature for 7 hours and then left standing overnight.

The reaction mixture is poured into glacial water containing sodium bicarbonate and extraction is carried out with ethyl acetate. After washing with water and drying, the solvent is removed by distillation and the residue is purified by chromatography using silica gel (benzene:ethyl acetate = 4:1). As a result, 0.90 g of crystals of 9α-fluoro-16β-methyl-1,4-pregnadiene-11β,17α,21-triol-3,20-dione 17α,21-ethyl ortho γ-ethoxycarbonyl butyrate is obtained (Melting point: 150—155°C).

To 0.90 g of 9α-fluoro-16β-methyl-1,4-pregnadiene-11β,17α,21-triol-3,20-dione 17α,21-ethyl ortho γ-ethoxycarbonyl butyrate are added 30 ml of ethanol and 1.35 ml of 0.28% aqueous solution of aluminum chloride and reaction is carried out at room temperature for 4 hours.

The reaction mixture is poured into saturated saline solution and extracted with ethyl acetate. After washing with water and drying, the solvent is removed by distillation to give 0.77 g of 9α-fluoro-16β-methyl-1,4-pregnadiene-11β,17α,21-triol-3,20-dione 17α-(γ-ethoxycarbonyl butyrate) as an amorphous solid.

To 0.60 g of 9α-fluoro-16β-methyl-1,4-pregnadiene-11β,17α,21-triol-3,20-dione 17α-(γ-ethoxycarbonyl butyrate) are added 12 ml of methylene chloride and 0.42 ml of triethylamine and 0.11 ml of methanesulfonyl chloride is further added thereto under ice-cooling. After 10 minutes, the mixture is restored to room temperature and stirred for 3 hours.

The reaction mixture is washed with 1N-hydrochloric acid, saturated aqueous solution of sodium hydrogen carbonate and saturated saline solution. After drying, the solvent is removed by distillation and 0.71 g of 9α-fluoro-16β-methyl-1,4-pregnadiene-11β,17α,21-triol-3,20-dione 17α-(γ-ethoxycarbonyl butyrate)21-methane sulfonate is obtained as an amorphous solid.

To 0.71 g of 9α-fluoro-16β-methyl-1,4-pregnadiene-11β,17α,21-triol-3,20-dione 17α-(γ-ethoxycarbonyl butyrate)21-methanesulfonate are added 5 ml of dimethylformamide and 0.33 g of lithium chloride and the mixture is stirred at 80°C for 12 hours.

After the solvent is removed by distillation under reduced pressure, methylene chloride is added to the residue and the mixture is thoroughly washed with water and dried. The solvent is removed by distillation and the residue is purified by chromatography using silica gel (benzene:ethyl acetate = 4:1). As the result, 0.55 g of 21-chloro-9α-fluoro-16β-methyl-1,4-pregnadiene-11β,17α-diol-3,20-dione 17α-(γ-ethoxycarbonyl butyrate) is obtained as an amorphous solid.

IR: 3450, 2950, 1735, 1665 $cm^{-1}$.

NMR ($CDCl_3$, δ): 1.02 (S, 18-$CH_3$), 1.57 (S, 19-$CH_3$), 1.23 and 4.10 (t and q, 17-$OCOCH_2CH_3$), 4.00 (S, 21-$CH_2$), 6.12 (S, 4-CH), 6.28 (d-d, 2-CH).

## Example 16

9α-fluoro-16β-methyl-1,4-pregnadiene-11β,17α,21-triol-3,20-dione 17α-(β-methoxycarbonyl propionate)21-propionate

To 0.80 g of 9α-fluoro-16β-methyl-1,4-pregnadiene-11β,17α,21-triol-3,20-dione 17α-(β-methoxycarbonyl propionate) obtained in Example 10 are added 8 ml of methylene chloride and 0.60 ml of triethylamine and 0.2 ml of propionyl chloride is further added thereto under ice-cooling. After 10 minutes, the mixture is restored to room temperature and stirred for further two hours.

50 ml of methylene chloride is added to the reaction mixture and the resulting mixture is washed with 1N-hydrochloric acid, saturated aqueous solution of sodium hydrogen carbonate and saturated saline

solution and dried. The solvent is removed by distillation and the residue is purified by chromatography using silica gel (benzene:ethyl acetate = 4:1). As the result, 0.57 g of 9α-fluoro-16β-methyl-1,4-pregnadiene-11β,17α,21-triol-3,20-dione 17α-(β-methoxycarbonyl propionate)21-propionate is obtained as an amorphous solid.

IR: 3470, 2960, 1740, 1670 $cm^{-1}$

NMR ($CDCl_3$, δ): 1.00 (S, 18-$CH_3$), 1.55 (S, 19-$CH_3$), 2.62 (S, 17-$OCOCH_2CH_2COO$—), 3.65 (S, 17-$COOCH_3$), 6.12 (S, 4-CH), 6.32 (d-d, 2-CH)

Example 17

21-chloro-9α-fluoro-16β-methyl-1,4-pregnadien-11β,17α-diol-3,20-dione 17α-(β-ethoxycarbonyl propionate)

To 500 mg of 21-chloro-9α-fluoro-16β-methyl-1,4-pregnadiene-11β,17α-diol-3,20-dione 17α-(β-methoxycarbonyl propionate) obtained in Example 12 is added a solution of 4,4 mg of metallic sodium in 10 ml of ethanol and the mixture is stirred at room temperature for 2.5 hours and then left standing overnight.

The reaction mixture is poured into saturated saline solution and extraction is carried out with ethyl acetate. After washing with water and drying, the solvent is removed by distillation and the residue is purified by chromatography using silica gel (benzene:ethyl acetate = 4:1). As the result, 360 mg of crystals of 21-chloro-9α-fluoro-16β-methyl-1,4-pregnadiene-11β,17α-diol-3,20-dione 17α-(β-ethoxycarbonyl propionate) are obtained. The crystals are recrystallized from ethyl acetate-n-hexane.

Melting point: 228—230°C

IR: 3460, 2950, 1740, 1670 $cm^{-1}$

NMR ($CDCl_3$, δ): 1.30 (S, 18-$CH_3$), 1.58 (s, 19-$CH_3$), 1.22 and 4.08 (t and q, 17-$COOCH_2CH_3$), 6.07 (S, 4-CH), 6.27 (d-d, 2-CH)

Experimental Example

[Method]

Experiments are conducted, according to the following procedures, on topical anti-inflammatory activity and on thymolytic action which is an index of systemic side effects, for the purpose of clarifying pharmacological activities of the compounds of the present invention.

1) Experiment on topical anti-inflammatory activity

Male mice of ddY-strain having a body weight of 15—20 g are divided at random into groups, each consisting of 10 mice.

0.9% sodium chloride, 0.4% Tween 80, 0.5% carboxymethyl cellulose and 0.9% benzyl alcohol are dissolved or suspended in distilled water, which is used as a suspending medium.

The compounds to be tested are dissolved in a mixture of the suspending medium:pyridine:diethyl ether = 1:4:5 and mixed with equal volume of diethyl ether containing 10% croton oil and the mixture is used as the test liquid.

Commercially available felt having a thickness of 5 mm is cut in 7 mm × 7 mm square and adhered to ring tweezers using alkyl-α-cyanoacrylate. The felt is soaked in the test liquid and the liquid is applied to the right ear of the mouse by rubbing it with the felt at a fixed pressure without anesthesia. The left ear is left untreated. At 5th hours after application, mice are killed and both of the ears are cut and weighed. The rate (%) of increase in the weight of right ear against that of left ear is calculated as the edema ratio. Edema ratios obtained on the test compounds are compared with those obtained on control and edema-repressing rate is obtained.

2) Experiment on thymolytic action

Male rats of Wistar strain having a body weight of 120—150 g are divided at random into groups, each consisting of 8 rats.

The compounds to be tested are dissolved in a liquid consisting of croton oil:cotton seed oil:ethanol = 1:89:10 and the solution is used for injection.

The rats are anesthesized by inhalation of ether and 20 ml of air are subcutaneously injected into hypodermic organ at the back of the rats using a thin injection needle to form an oval-shaped air cavity. After the rats recovered from anesthesia they are kept on normal food and water. At 8th days after injection, rats are killed by depletion and dissected. The thymus gland is taken out and the wet weight is measured. Thymus weights obtained on the test compounds are divided with those obtained on control, and thymus gland atrophy rate is obtained.

[Results]

In each of the experiments on anti-inflammatory activity and thymolytic action, clobetasol 17-propionate and betamethasone 17,21-dipropionate are used standard compounds and anti-inflammatory activity ratio and thymolytic activity ratio against clobetasol 17-propionate are calculated using linear regression parallel test method.

The results are shown in Table 1 below. In the table, the compounds are indicated by relevant Example

numbers or Comparative Example numbers.

TABLE 1

| Compounds | Anti-inflammatory activity ratio (A) | Thymolytic activity ratio (B) | R=A/B |
|---|---|---|---|
| Clobetasol 17-propionate | 1 | 1 | 1 |
| Example 2 | 3.0 | 0.018 | 167 |
| Example 4 | 1.3 | 0.048 | 27 |
| Example 6 | 0.74 | 0.24 | 3.1 |
| Example 8 | 0.51 | 0.092 | 5.5 |
| Example 12 | 1.3 | 0.072 | 18 |
| Example 13 | 1.0 | <0.01 | >100 |
| Example 14 | 0.2 | <0.01 | >20 |
| Example 15 | 0.68 | 0.012 | 57 |
| Example 17 | 0.56 | 0.007 | 80 |
| Comparative Example 1 | 0.53 | 0.29 | 1.8 |
| Comparative Example 2 | 0.96 | 1.2 | 0.8 |
| Betamethasone 17,21-dipropionate | 0.080 | 0.030 | 2.7 |

As is apparent from the above Table 1, the compounds of the present invention show an anti-inflammatory activity comparable to or better than that of clobetasol 17-propionate.

With respect to thymolytic action, all the compounds of the present invention have weaker action than clobetasol 17-propionate.

Thus, since the present compounds have very weak systemic side effects and strong topical anti-inflammatory activity, it is obvious that they are useful compounds.

**Claims**

1. Corticoid derivatives represented by the general formula (I):

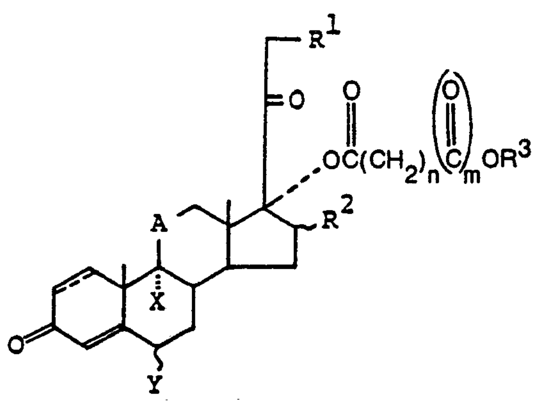

(I)

[wherein A is a group

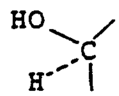 or 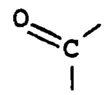 ;

X is a hydrogen atom or halogen atom; Y is a hydrogen atom, oxo group, or α- or β-halogen atom, hydroxyl group or alkyl group having 1 to 10 carbon atoms; m is 0 or 1; n is 0 or an integer of 2 to 5; $R^1$ is a hydroxyl group, halogen atom, a group represented by the formula (II):

$$—OSO_2R^4 \qquad (II)$$

(wherein $R^4$ is an alkyl group or halogenated alkyl group having 1 to 10 carbon atoms) or a group represented by the formula (III):

$$—OCOR^5 \qquad (III)$$

(wherein $R^5$ is an alkyl or halogenated alkyl group having 1 to 10 carbon atoms); $R^2$ is a hydrogen atom or alkyl group having 1 to 10 carbon atoms at the α- or β-positions; $R_3$ is an alkyl group having 1 to 10 carbon atoms; and the bond between $C_1$ and $C_2$ is a single bond or double bond, provided where m is 0, n is 0 and Y is an oxo group and where m is 1, n is an integer of 2 to 5].

2. Corticoid derivatives according to claim 1 represented by the general formula:

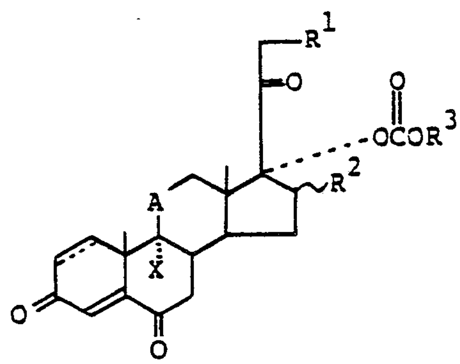

(wherein A, X, $R^1$, $R^2$ and $R^3$ are as defined in claim 1; and the bond between $C_1$ and $C_2$ is a single bond or double bond).

3. Corticoid derivatives according to claim 1 represented by the general formula:

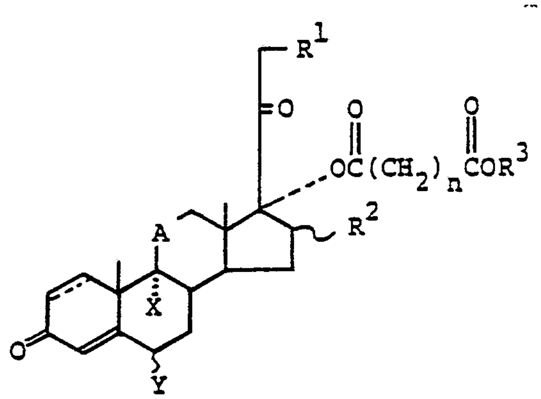

(wherein A, X, Y, n, $R^1$, $R^2$ and $R^3$ are as defined in claim 1 and the bond between $C_1$ and $C_2$ is a single bond or double bond).

4. 9α-Fluoro-16β-methyl-1,4-pregnadiene-11β,17α,21-triol-3,6,20-trione 17α-methyl carbonate.

5. 21-Chloro-9α-fluoro-16β-methyl-1,4-pregnadiene-11β,17α-diol-3,6,20-trione 17α-methyl carbonate.

6. 9α-Fluoro-16β-methyl-1,4-pregnadiene-11β,17α,21-triol-3,6,20-trione 17α-ethyl carbonate.

7. 21-Chloro-9α-fluoro-16β-methyl-1,4-pregnadiene-11β,17α-diol-3,6,20-trione 17α-ethyl carbonate.

8. 9α-Fluoro-16β-methyl-1,4-pregnadiene-11β,17α,21-triol-3,6,20-trione 17α-propyl carbonate.

9. 21-Chloro-9α-fluoro-16β-methyl-1,4-pregnadiene-11β,17α-diol-3,6,20-trione 17α-propyl carbonate.

10. 9α-Fluoro-16α-methyl-1,4-pregnadiene-11β,17α,21-triol-3,6,20-trione 17α-methyl carbonate.

11. 21-Chloro-9α-Fluoro-16α-methyl-1,4-pregnadiene-11β,17α-diol-3,6,20-trione 17α-methyl carbonate.

12. 4-Pregnene-11β,17α,21-triol-3,20-dione 17α-(β-methoxycarbonyl propionate).

13. 9α-Fluoro-16β-methyl-1,4-pregnadiene-11β,17α,21-triol-3,20-dione 17α-(β-methoxycarbonyl propionate).

14. 9α-Fluoro-16β-methyl-1,4-pregnadiene-11β,17α,21-triol-3,6,20-trione 17α-(β-methoxycarbonyl propionate).

15. 21-Chloro-9α-fluoro-16β-methyl-1,4-pregnadiene-11β,17α-diol-3,20-dione 17α-(β-methoxycarbonyl propionate).

16. 21-Chloro-9α-fluoro-16β-methyl-1,4-pregnadiene-11β,17α-diol-3,6,20-trione 17α-(β-methoxycarbonyl propionate).

17. 21-Chloro-9α-fluoro-16α-methyl-1,4-pregnadiene-11β,17α-diol-3,20-dione 17α-(β-methoxycarbonyl propionate).

18. 21-Chloro-9α-fluoro-16β-methyl-1,4-pregnadiene-11β,17α-diol-3,20-dione 17α-(γ-ethoxycarbonyl butyrate).

19. 9α-Fluoro-16β-methyl-1,4-pregnadiene-11β,17α,21-triol-3,20-dione 17α-(β-methoxycarbonyl propionate) 21-propionate.

20. 21-Chloro-9α-Fluoro-16β-methyl-1,4-pregnadiene-11β,17α-diol-3,20-dione 17α-(β-ethoxycarbonyl propionate).

21. A process for preparing the corticoid derivatives according to claim 1, characterized by reacting a compound represented by the general formula (IV):

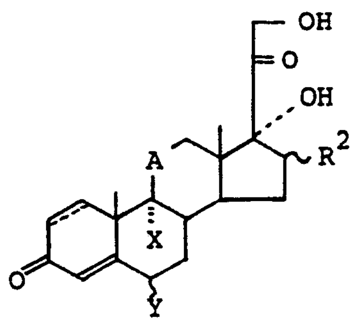

(IV)

(wherein A, X, Y and $R^2$ are as defined in claim 1) with an orthoester derivative represented by the general formula (V):

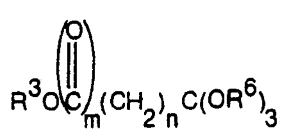

(V)

(wherein m, n and $R^3$ are as defined in claim 1 and $R^6$ is an alkyl group having 1 to 10 carbon atoms, provided where m is 0, n is 0 and where m is 1, n is 0 or an integer of 2 to 5), to obtain a compound represented by the general formula (VI):

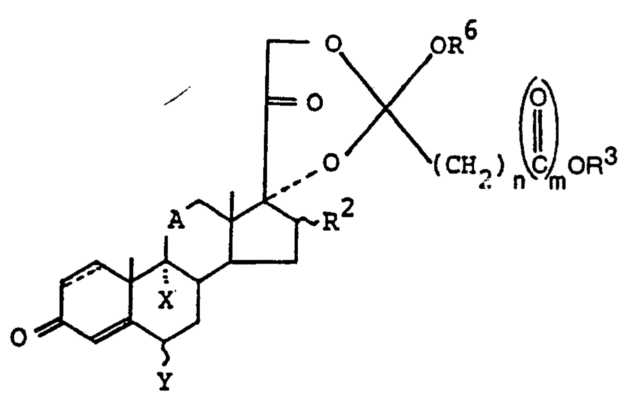

(VI)

(wherein A, X, Y, m, n, $R^2$, $R^3$ and $R^6$ are as defined above) and hydrolyzing said compound represented by

the general formula (VI) to obtain a compound represented by the general formula (Ia):

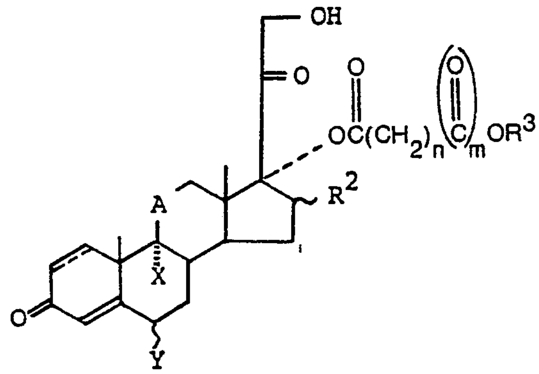

(Ia)

(wherein A, X, Y, $R^2$, m, n and $R^3$ have the same significance as defined above) and, if desired, reacting said compound represented by the general formula (Ia)

a) either with a sulfonic acid derivative represented by the general formula (IIa) or (IIb):

$$(R^4SO_2)_2O \quad \text{(IIa)}$$

$$R^4SO_2X^1 \quad \text{(IIb)}$$

(wherein $R^4$ is as defined in claim 1 and $X^1$ is a halogen atom) to obtain a compound represented by the general formula I, wherein $R^1$ is a group represented by the formula (II) as defined above; or

b) with a carboxylic acid derivative represented by the general formula (IIIa) or (IIIb):

$$(R^5CO)_2O \quad \text{(IIIa)}$$

$$R^5COX^1 \quad \text{(IIIb)}$$

(wherein $R^5$ is as defined in claim 1 and $X^1$ is a halogen atom) to obtain a compound represented by the general formula (I), wherein $R^1$ is a group represented by the formula (III) as defined above; or

c) reacting the compound represented by the general formula (I) wherein $R^1$ is a group represented by the formula (II) (as defined above), as obtained in the reaction a) above with a halogen ion donor to obtain a compound represented by the general formula (I) wherein $R^1$ is a halogen atom.

22. A process for producing corticoid 17α,21-cyclic ortho ester derivatives represented by the formula (VI):

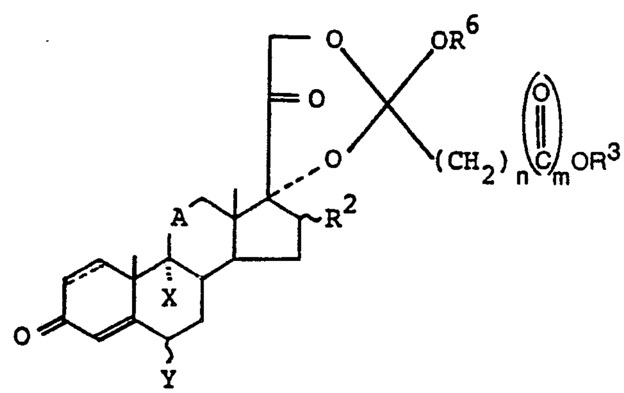

(VI)

(wherein A, X, Y, m, n, $R^2$ and $R^3$ are as defined in claim 1; $R^6$ is an alkyl group having 1 to 10 carbon atoms; and the bond between $C_1$ and $C_2$ is a single bond or double bond, which comprises reacting a compound represented by the general formula (IV):

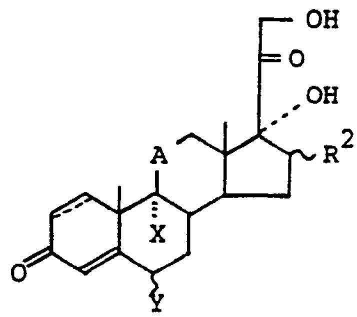

(IV)

[wherein A, X, Y and $R^2$ have the same significance as defined in the general formula (VI)] with an orthoester derivative represented by the general formula (V):

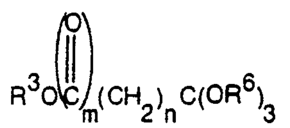

(V)

[wherein m, n, $R^3$ and $R^6$ have the same significance as defined in the general formula (VI), provided where m is 0, n is 0 and where m is 1, n is 0 or an integer of 2 to 5].

23. A process for producing corticoid derivatives represented by the general formula (Ia):

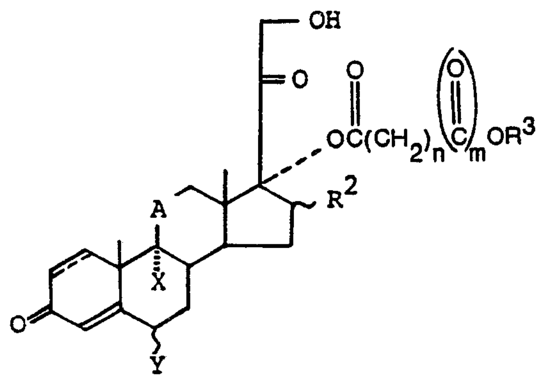

(Ia)

(wherein A, X, Y, m, n, $R^2$ and $R^3$ are as defined in claim 1; and the bond between $C_1$ and $C_2$ is a single bond or double bond), which comprises hydrolyzing a compound represented by the general formula (VI):

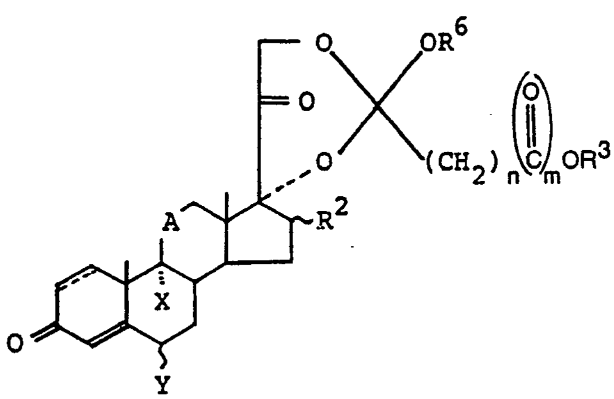

(VI)

(wherein A, X, Y, n, m, $R^2$ and $R^3$ have the same significance as defined in the general formula (Ia) and $R^6$ is an alkyl group having 1 to 10 carbon atoms).

24. A process for producing corticoid derivatives represented by the general formula (Ib):

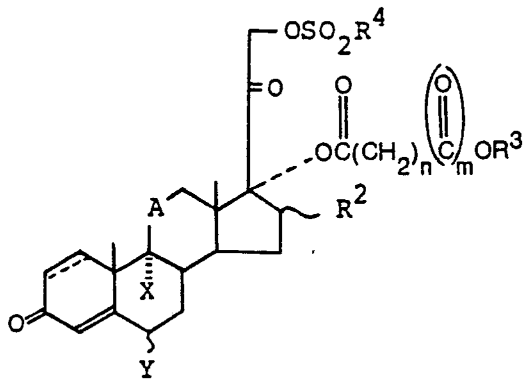

(Ib)

(wherein A, X, Y, m, n, $R^2$, $R^3$ and $R^4$ are as defined in claim 1, and the bond between $C_1$ and $C_2$ is a single bond or double bond), which comprises reacting a compound represented by the general formula (Ia):

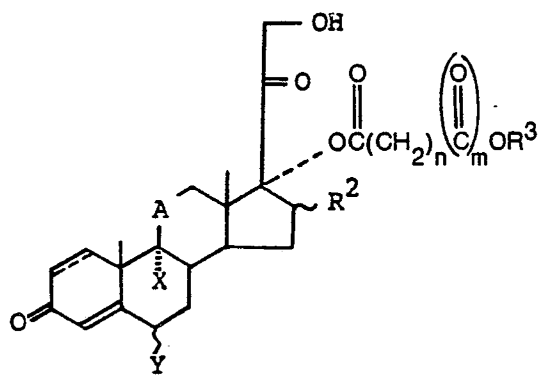

(Ia)

(wherein A, X, Y, $R^2$, m, n and $R^3$ have the same significance as defined in the general formula (Ib)) with a sulfonic acid derivative represented by the general formula (IIa) or (IIb):

$(R^4SO_2)_2O$ (IIa)

$R^4SO_2X^1$ (IIb)

[wherein $R^4$ has the same significance as defined in the general formula (Ib) and $X^1$ is a halogen atom].

25. A process for producing corticoid derivatives represented by the general formula (Ic):

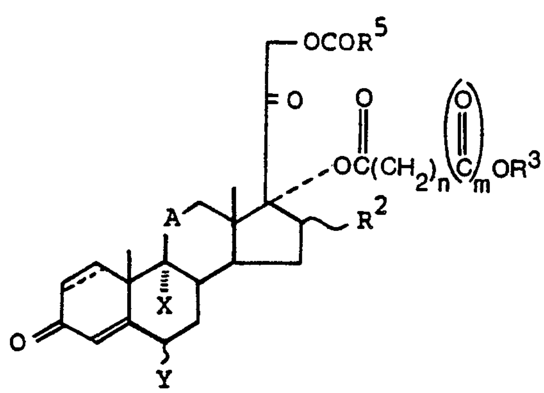

(Ic)

(wherein A, X, Y, m, n, $R^2$, $R^3$ and $R^5$ are as defined in claim 1; and the bond between $C_1$ and $C_2$ is a single bond or double bond), which comprises reacting a compound represented by the general formula (Ia):

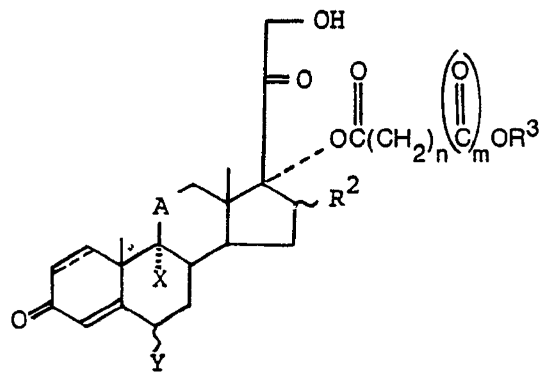

(Ia)

[wherein A, X, Y, $R^2$, m, n and $R^3$ have the same significance as defined in the general formula (Ic)] with a carboxylic acid derivative represented by the general formula (IIIa) or (IIIb):

$$(R^5CO)_2O \qquad \text{(IIIa)}$$

$$R^5COX^1 \qquad \text{(IIIb)}$$

[wherein $R^5$ has the same significance as defined in the general formula (Ic) and $X^1$ is a halogen atom].

26. A process for producing corticoid derivatives represented by the general formula (Id):

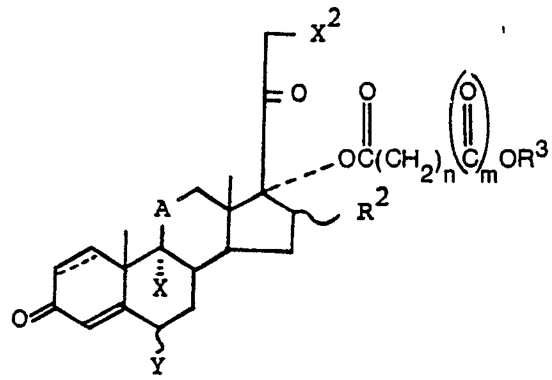

(Id).

(wherein A, X, Y, m, n, $R^2$ nd $R^3$ are as defined in claim 1; $X^2$ is a halogen atom; and the bond between $C_1$ and $C_2$ is a single bond or double bond), which comprises reacting a compound represented by the general formula (Ib):

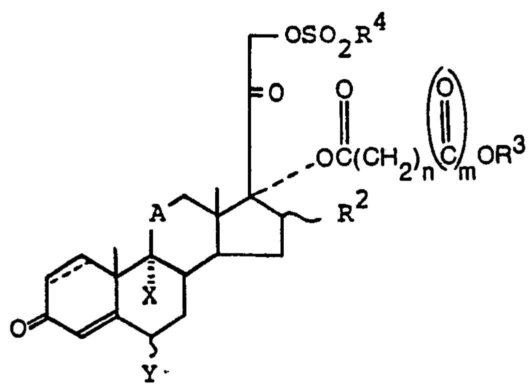

(Ib)

[wherein A, X, Y, $R^2$, m, n and $R^3$ have the same significance as defined in the general formula (Id) and $R^4$ is an alkyl group or halogenated alkyl group having 1 to 10 carbon atoms] with a halogen ion donor.

27. A process for producing corticoid derivatives represented by the general formula (Id)':

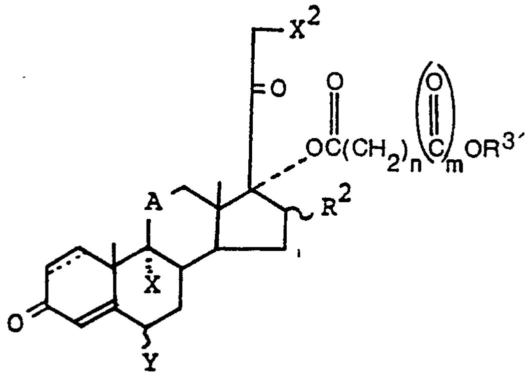

(Id)'

(wherein A, X, Y, m, n and $R^2$ are as defined in claim 1; $X^2$ is a halogen atom; $R^{3'}$ is an alkyl group having 1 to 10 carbon atoms; and the bond between $C_1$ and $C_2$ is a single bond or double bond), which comprises reacting a compound represented by the general formula (Id):

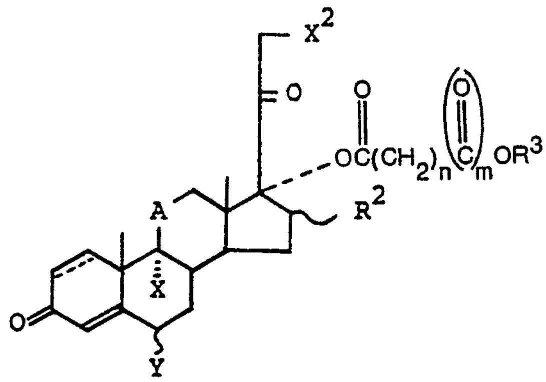

(Id)

[wherein A, X, Y, $R^2$, m, n and $X^2$ have the same significance as defined in the general formula (Id)' and $R^3$ is an alkyl group having 1 to 10 carbon atoms but is different from $R^{3'}$ of the general formula (Id)'] with an alcohol represented by the general formula (VIII):

$$R^{3'}—OH \quad \text{(VIII)}$$

[wherein $R^{3'}$ has the same significance as defined in the general formula (Id)'] to effect interesterification.

28. A pharmaceutical composition consisting of an effective amount of corticoid derivatives according to claim 1.

**Patentansprüche**

1. Corticoid-Derivate der allgemeinen Formel (I)

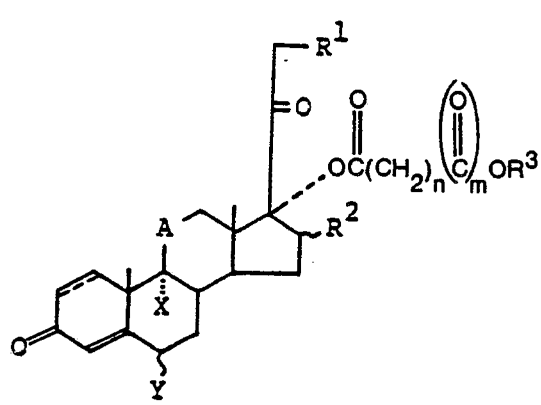

(I)

worin A eine Gruppe

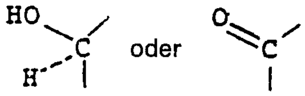

ist,

X für ein Wasserstoffatom oder ein Halogenatom steht, Y ein Wasserstoffatom, eine Oxogruppe oder ein α- oder ein β-Halogenatom, eine Hydroxy- oder eine Alkylgruppe mit 1 bis 10 Kohlenstoffatomen ist, m 0 oder 1 ist, n 0 ist oder eine ganze Zahl von 2 bis 5 bedeutet, $R^1$ für eine Hydroxygruppe, ein Halogenatom, eine Gruppe der allgemeinen Formel (II):

$$—OSO_2R^4 \qquad (II)$$

worin $R^4$ eine Alkylgrupe oder eine halogenierte Alkylgruppe mit 1 bis 10 Kohlenstoffatomen ist, oder eine Gruppe der allgemeinen Formel (III):

$$—OCOR^5 \qquad (III)$$

worin $R^5$ eine Alkylgruppe oder eine halogenierte Alkylgruppe mit 1 bis 10 Kohlenstoffatomen ist, $R^2$ für ein Wasserstoffatom oder eine Alkylgruppe mit 1 bis 10 Kohlenstoffatomen in der α- oder β-Position, $R^3$ eine Alkylgruppe mit 1 bis 10 Kohlenstoffatomen ist und die Bindung zwischen den Kohlenstoffatomen $C_1$ und $C_2$ eine Einfachbindung oder Doppelbindung ist, mit der Maßgabe, daß n 0 ist und Y für eine Oxogruppe steht, wenn m gleich 0 ist, und n eine ganze Zahl von 2 bis 5 ist, wenn m 1 bedeutet.

2. Corticoid-Derivate nach Anspruch 1 gemäß der allgemeinen Formel:

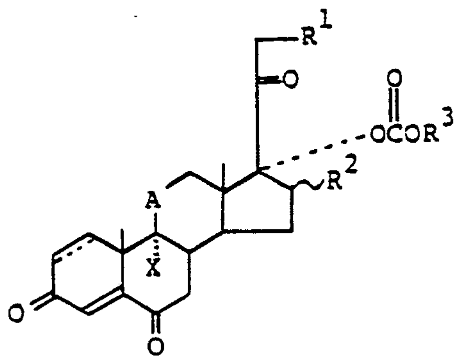

worin A, X, $R^1$, $R^2$ und $R^3$ die in Anspruch 1 definierte Bedeutung haben und die Bindung zwischen den Kohlenstoffatomen $C_1$ und $C_2$ eine Einfachbindung oder Doppelbindung ist.

3. Corticoid-Derivate nach Anspruch 1 entsprechend der allgemeinen Formel:

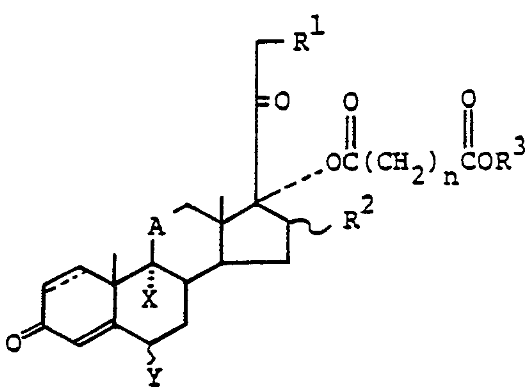

worin A, X, Y, n, $R^1$, $R^2$ und $R^3$ die in Anspruch 1 definierte Bedeutung haben und die Bindung zwischen den Kohlenstoffatomen $C_1$ und $C_2$ eine Einfachbindung oder eine Doppelbindung ist.

4. 9α-Fluor-16β-methyl-1,4-pregnadien-11β,17α,21-triol-3,6,20-trion-17α-methylcarbonat.

5. 21-Chlor-9α-fluor-16β-methyl-1,4-pregnadien-11β,17α-diol-3,6,20-trion-17α-methylcarbonat.

6. 9α-Fluor-16β-methyl-1,4-pregnadien-11β,17α,21-triol-3,6,20-trion-17α-ethylcarbonat.

7. 21-Chlor-9α-fluor-16β-methyl-1,4-pregnadien-11β,17α-diol-3,6,20-trion-17α-ethylcarbonat.

8. 9α-Fluor-16β-methyl-1,4-pregnadien-11β,17α,21-triol-3,6,20-trion-17α-propylcarbonat.

9. 21-Chlor-9α-fluor-16β-methyl-1,4-pregnadien-11β,17α-diol-3,6,20-trion-17α-propylcarbonat.

10. 9α-Fluor-16α-methyl-1,4-pregnadien-11β,17α,21-triol-3,6,20-trion-17α-methylcarbonat.

11. 21-Chlor-9α-fluor-16α-methyl-1,4-pregnadien-11β,17α-diol-3,6,20-trion-17α-methylcarbonat.

12. 4-Pregnen-11β,17α,21-triol-3,20-dion-17α-(β-methoxycarbonylpropionat).

13. 9α-Fluor-16β-methyl-1,4-pregnadien-11β,17α,21-triol-3,20-dion-17α-(β-methoxycarbonyl-propionat).

14. 9α-Fluor-16β-methyl-1,4-pregnadien-11β,17α,21-triol-3,6,20-trion-17α-(β-methoxycarbonyl-propionat).

15. 21-Chlor-9α-fluor-16β-methyl-1,4-pregnadien-11β,17α-diol-3,20-dion-17α-(β-methoxycarbonyl-propionat).

16. 21-Chlor-9α-fluor-16β-methyl-1,4-pregnadien-11β,17α-diol-3,6,20-trion-17α-(β-methoxycarbonyl-propionat).

17. 21-Chlor-9α-fluor-16α-methyl-1,4-pregnadien-11β,17α-diol-3,20-dion-17α-(β-methoxycarbonyl-propionat).

18. 21-Chlor-9α-fluor-16β-methyl-1,4-pregnadien-11β,17α-diol-3,20-dion-17α-(γ-ethoxycarbonyl-butyrat).

19. 9α-Fluor-16β-methyl-1,4-pregnadien-11β,17α,21-triol-3,20-dion-17α-(β-methoxycarbonyl-propionat)-21-propionat.

20. 21-Chlor-9α-fluor-16β-methyl-1,4-pregnadien-11β,17α-diol-3,20-dion-17α-(β-ethoxycarbonyl-propionat).

21. Verfahren zur Herstellung der Corticoid-Derivate nach Anspruch 1, dadurch gekennzeichnet, daß man eine Verbindung der allgemeinen Formel (IV)

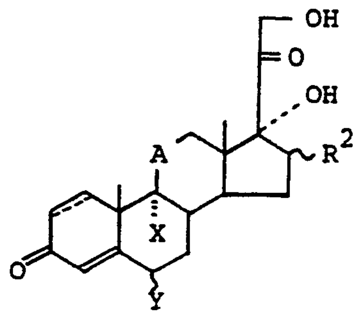

(IV)

worin A, X, Y und $R^2$ die in Anspruch 1 definierte Bedeutung haben, mit einem ortho-Ester-Derivat der allgemeinen Formel (V)

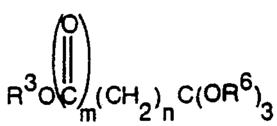

(V)

worin m, n und $R^3$ die in Anspruch 1 definierte Bedeutung haben und $R^6$ eine Alkylgruppe mit 1 bis 10 Kohlenstoffatomen ist, mit der Maßgabe, daß n 0 ist, wenn m 0 ist, und daß n 0 oder eine ganze Zahl von 2 bis 5 ist, wenn m 1 ist, unter Erhalt einer Verbindung der allgemeinen Formel (VI) umsetzt,

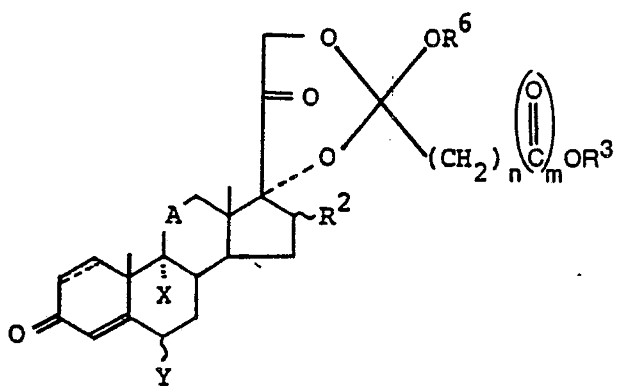

(VI)

worin A, X, Y, m, n, $R^2$, $R^3$ und $R^6$ die oben definierte Bedeutung haben und die durch die allgemeine Formel (VI) wiedergegebene Verbindung unter Erhalt einer Verbindung der allgemeinen Formel (Ia) hydrolysiert

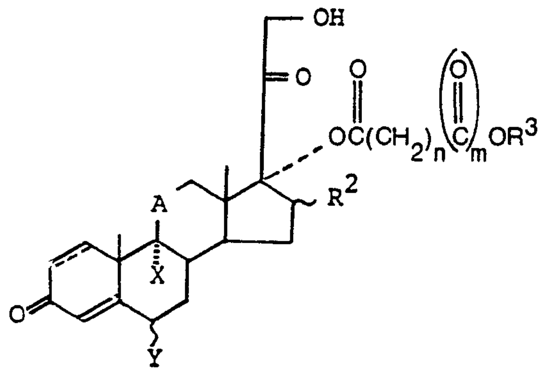

(Ia)

worin A, X, Y, $R^2$, m, n und $R^3$ dieselbe Bedeutung haben, wie sie oben angegeben wurde, und — sofern erwünscht — die durch die allgemeine Formel (Ia) wiedergegebene Verbindung

a) entweder mit einem Sulfonsäure-Derivat der allgemeinen Formel (IIa) oder der allgemeinen Formel (IIb) umsetzt:

$$(R^4SO_2)_2O \qquad \text{(IIa)}$$

$$R^4SO_2X^1 \qquad \text{(IIb)}$$

worin $R^4$ die in Anspruch 1 angegebene Bedeutung hat und $X^1$ ein Halogenatom ist, wobei man eine Verbindung der allgemeinen Formel (I) erhält, worin $R^1$ eine Gruppe der oben angegebenen allgemeinen Formel (II) ist,

b) oder mit einem Carbonsäure-Derivat der allgemeinen Formel (IIIa) oder der allgemeinen Formel (IIIb) umsetzt:

$$(R^5CO)_2O \qquad \text{(IIIa)}$$

$$R^5COX^1 \qquad \text{(IIIb)}$$

worin $R^5$ die in Anspruch 1 definierte Bedeutung hat und $X^1$ ein Halogenatom ist, wobei man eine Verbindung der allgemeinen Formel (I) erhält, worin $R^1$ eine durch die oben angegebene Formel (III) definierte Gruppe ist,

c) oder die Verbindung der allgemeinen Formel (I), worin $R^1$ eine Gruppe der oben definierten Formel (II) ist, wie sie im oben beschriebenen Reaktionsschritt (a) erhalten wurde mit einem Halogenionen-Donor unter Erhalt einer Verbindung der allgemeinen Formel (I) umsetzt, worin $R^1$ ein Halogenatom ist.

22. Verfahren zur Herstellung von die Positionen 17 und 21 einschließenden zyklischen Corticoid-ortho-Ester-Derivaten der Formel (VI):

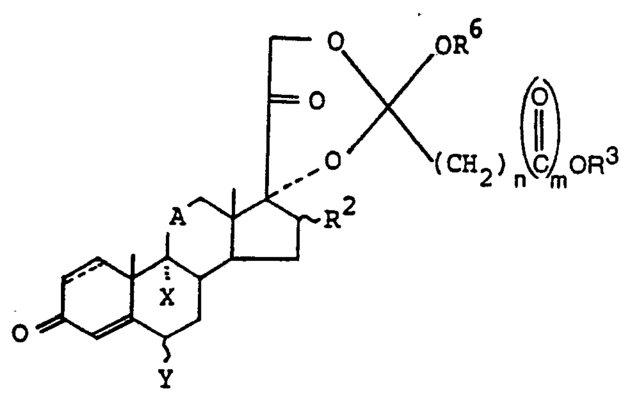

(VI)

worin A, X, Y, m, n, $R^2$ und $R^3$ die in Anspruch 1 definierte Bedeutung haben, $R^6$ eine Alkylgruppe mit 1 bis 10 Kohlenstoffatomen und die Bindung zwischen den Kohlenstoffatomen $C_1$ und $C_2$ eine Einfachbindung oder Doppelbindung ist, das die Umsetzung einer Verbindung der allgemeinen Formel (IV):

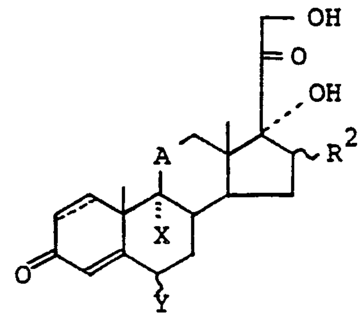

(IV)

worin A, X, Y und $R^2$ dieselbe Bedeutung haben, wie sie in Zusammenhang mit der allgemeinen Formel (VI) definiert wurde, mit einem ortho-Ester-Derivat der allgemeinen Formel (V) umfaßt:

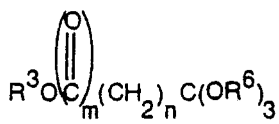

(V)

worin m, n, $R^3$ und $R^6$ dieselbe Bedeutung haben, wie sie in Zusammenhang mit der allgemeinen Formel (VI) definiert wurde, mit der Maßgabe, daß n 0 ist, wenn m 0 ist, und daß n 0 oder eine ganze Zahl von 2 bis 5 ist, wenn m 1 ist.

23. Verfahren zur Herstellung von Corticoid-Derivaten der allgemeinen Formel (Ia):

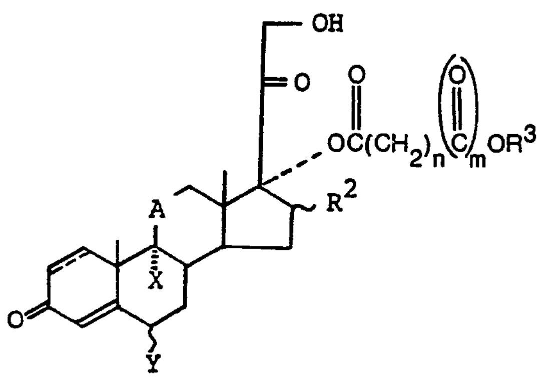

(Ia)

worin A, X, Y, m, n, $R^2$ und $R^3$ die in Anspruch 1 definierte Bedeutung haben und die Bindung zwischen den Kohlenstoffatomen $C_1$ und $C_2$ eine Einfachbindung oder Doppelbindung ist, das die Hydrolyse einer Verbindung der allgemeinen Formel (VI) umfaßt:

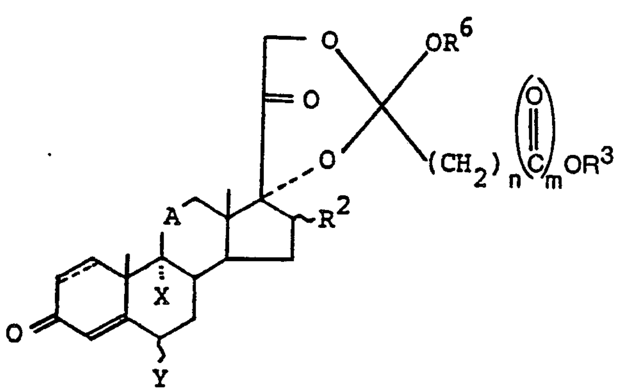

(VI)

worin A, X, Y, n, m, $R^2$ und $R^3$ dieselbe Bedeutung haben, wie sie in Zusammenhang mit der allgemeinen Formel (Ia) definiert wurde, und $R^6$ eine Alkylgruppe mit 1 bis 10 Kohlenstoffatomen ist.

24. Verfahren zur Herstellung von Corticoid-Derivaten der allgemeinen Formel (Ib):

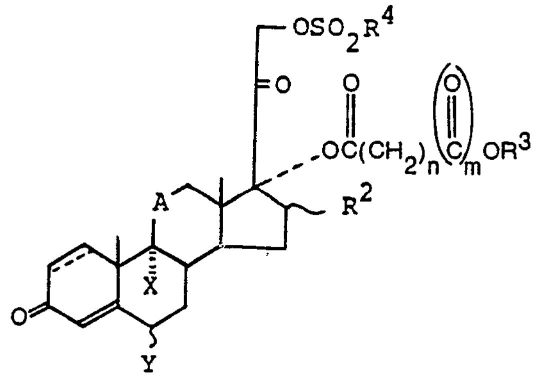

(Ib)

worin A, X, Y, m, n, $R^2$, $R^3$ und $R^4$ die in Anspruch 1 definierte Bedeutung haben und die Bindung zwischen den Kohlenstoffatomen $C_1$ und $C_2$ eine Einfachbindung oder Doppelbindung ist, das die Umsetzung einer Verbindung der allgemeinen Formel (Ia):

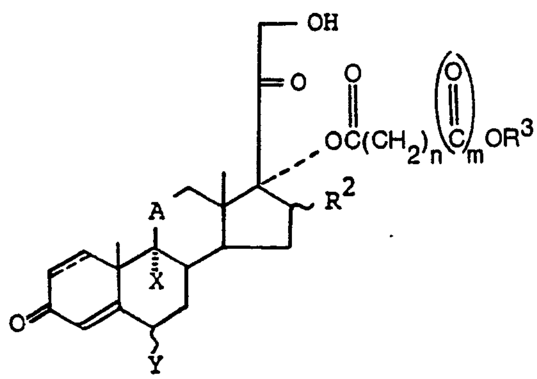

(Ia)

worin A, X, Y, $R^2$, m, n und $R^3$ dieselbe Bedeutung haben, wie sie in Zusammenhang mit der allgemeinen Formel (Ib) definiert wurde, mit einem Sulfonsäure-Derivat der allgemeinen Formel (IIa) oder der allgemeinen Formel (IIb) umfaßt:

$(R^4SO_2)_2O$ (IIa)

$R^4SO_2X^1$ (IIb)

worin $R^4$ dieselbe Bedeutung hat, wie sie in Zusammenhang mit der allgemeinen Formel (Ib) definiert wurde, und $X^1$ ein Halogenatom ist.

25. Verfahren zur Herstellung von Corticoid-Derivaten der allgemeinen Formel (Ic):

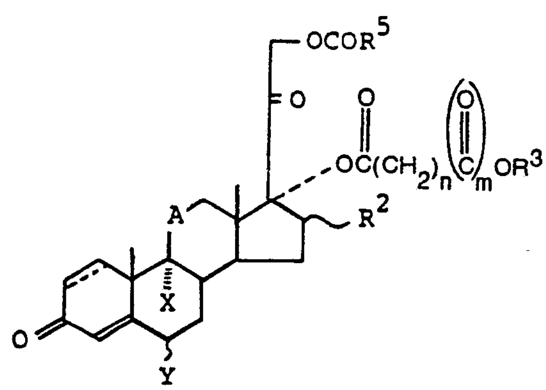

(Ic)

worin A, X, Y, m, n, $R^2$, $R^3$ und $R^5$ die in Anspruch 1 definierte Bedeutung haben und die Bindung zwischen

den Kohlenstoffatomen $C_1$ und $C_2$ eine Einfachbindung oder Doppelbindung ist, das die Umsetzung einer Verbindung der allgemeinen Formel (Ia):

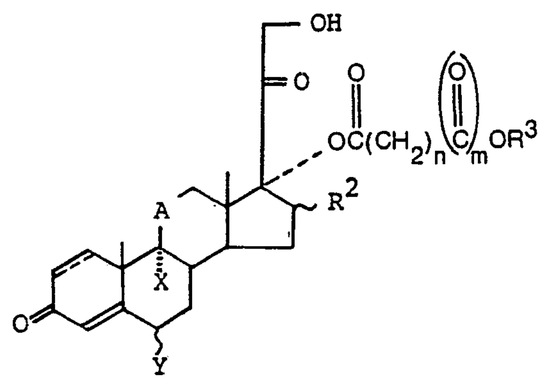

(Ia)

worin A, X, Y, $R^2$, m, n und $R^3$ dieselbe Bedeutung haben, wie sie in Zusammenhang mit der allgemeinen Formel (Ic) angegeben wurde, mit einem Carbonsäure-Derivat der allgemeinen Formel (IIIa) oder der allgemeinen Formel (IIIb) umfaßt:

$$(R^5CO)_2O \qquad \text{(IIIa)}$$

$$R^5COX^1 \qquad \text{(IIIb)}$$

worin $R^5$ dieselbe Bedeutung hat, wie sie in Zusammenhang mit der allgemeinen Formel (Ic) angegeben wurde und $X^1$ ein Halogenatom ist.

26. Verfahren zur Herstellung von Corticoid-Derivaten der allgemeinen Formel (Id):

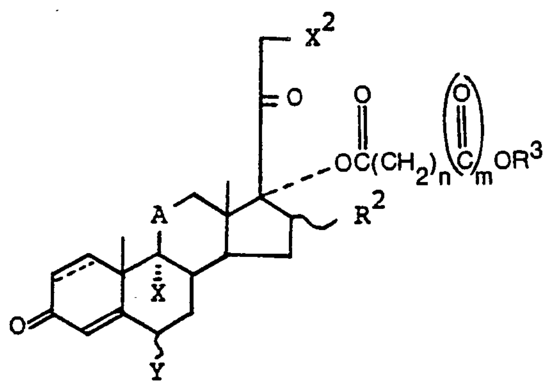

(Id)

worin A, X, Y, m, n, $R^2$ und $R^3$ die in Anspruch 1 definierte Bedeutung haben, $X^2$ ein Halogenatom ist und die Bindung zwischen den Kohlenstoffatomen $C_1$ und $C_2$ eine Einfachbindung oder Doppelbindung ist, das die Umsetzung einer Verbindung der allgemeinen Formel (Ib):

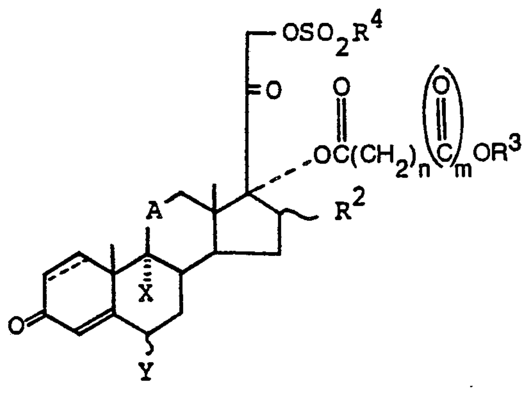

(Ib)

worin A, X, Y, $R^2$, m, n und $R^3$ dieselbe Bedeutung haben, wie sie in Zusammenhang mit der allgemeinen Formel (Id) definiert wurde, und $R^4$ eine Alkylgruppe oder eine halogenierte Alkylgruppe mit 1 bis 10 Kohlenstoffatomen ist, mit einem Halogenionen-Donor umfaßt.

27. Verfahren zur Herstellung von Corticoid-Derivaten der allgemeinen Formel (Id)':

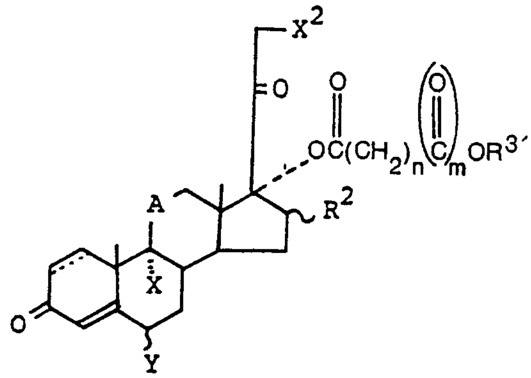

(Id)'

worin A, X, Y, m, n und $R^2$ die in Anspruch 1 definierte Bedeutung haben, $X^2$ ein Halogenatom ist, $R^{3'}$ eine Alkylgruppe mit 1 bis 10 Kohlenstoffatomen ist und die Bindung zwischen den Kohlenstoffatomen $C_1$ und $C_2$ eine Einfachbindung oder Doppelbindung ist, das die Umsetzung einer Verbindung der allgemeinen Formel (Id):

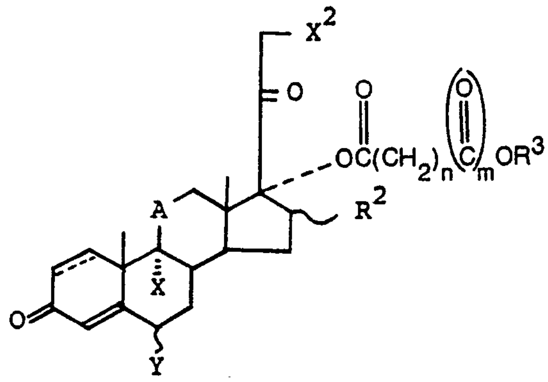

(Id)

worin A, X, Y, $R^2$, m, n und $X^2$ dieselbe Bedeutung haben, wie sie in Zusammenhang mit der allgemeinen Formel (Id)' definiert wurde und $R^3$ eine Alkylgruppe mit 1 bis 10 Kohlenstoffatomen ist, sich aber von dem Rest $R^{3'}$ der allgemeinen Formel (Id)' unterscheidet, mit einem Alkohol der allgemeinen Formel (VIII) umfaßt:

$$R^{3'}—OH \qquad (VIII)$$

worin $R^{3'}$ dieselbe Bedeutung hat, wie sie in Zusammenhang mit der allgemeinen Formel (Id)' definiert wurde, um eine Umesterung zu bewirken.

28. Pharmazeutische Zusammensetzung, bestehend aus einer wirksamen Menge von Corticoid-Derivaten nach Anspruch 1.

**Revendications**

1. Corticoïdes représentés par la formule générale (I):

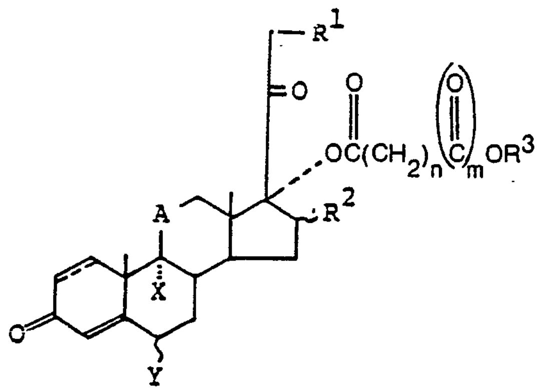

(I)

[dans laquelle:

A est un groupe

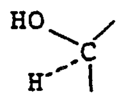 ou 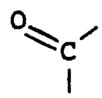 ;

X est un atome d'hydrogène ou un atome d'halogène;

Y est un atome d'hydrogène, un groupe oxo, ou un atome d'halogène, un groupe hydroxyle ou un groupe alkyle ayant de 1 à 10 atomes de carbone, en position α ou β;

n vaut 0 ou 1;

n vaut 0 ou un nombre entier de 2 à 5;

$R^1$ est un groupe hydroxyle, un atome d'halogène, un groupe représenté par la formule (II):

$$-OSO_2R^4 \qquad (II)$$

(dans laquelle $R^4$ est un groupe alkyle ou un groupe alkyle halogéné ayant de 1 à 10 atomes de carbone) ou un groupe représenté par la formule (III):

$$-OCOR^5 \qquad (III)$$

(dans laquelle $R^5$ est un groupe alkyle ou un groupe alkyle halogéné ayant de 1 à 10 atomes de carbone);

$R^2$ est un atome d'hydrogène ou un groupe alkyle ayant de 1 à 10 atomes de carbone en position α ou β;

$R^3$ est un groupe alkyle ayant de 1 à 10 atomes de carbone; et

la liaison entre $C_1$ et $C_2$ est une simple liaison ou une double liaison,

à la condition que, si m vaut 0, n vaut 0 et Y est un groupe oxo, et si m vaut 1, n est un nombre entier de 2 à 5].

2. Corticoïdes selon la revendication 1, représentés par la formule générale:

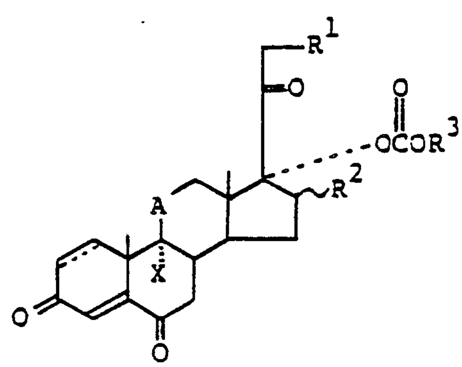

(dans laquelle:

A, X, $R^1$, $R^2$ et $R^3$ sont tels que définis à la revendication 1; et

la liaison entre $C_1$ et $C_2$ est une simple liaison ou une double liaison).

3. Corticoïdes selon la revendication 1, représentés par la formule générale:

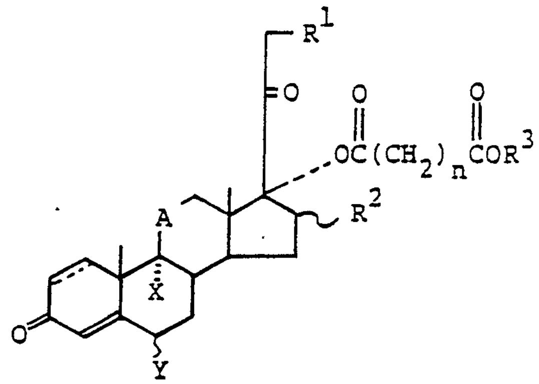

(dans laquelle:

A, X, Y, n, $R^1$, $R^2$ et $R^3$ sont tels que définis à la revendication 1; et

la liaison entre $C_1$ et $C_2$ est une simple liaison ou une double liaison).

4. (Méthyl carbonate)-17α de fluoro-9α méthyl-16β pregnadiène-1,4 triol-11β,17α,21 trione-3,6,20.

5. (Méthyl carbonate)-17α de chloro-21 fluoro-9α méthyl-16β pregnadiène-1,4 diol-11β,17α trione-3,6,20.

6. (Ethyl carbonate)-17α de fluoro-9α méthyl-16β pregnadiène-1,4 triol-11β,17α,21 trione-3,6,20.

7. (Ethyl carbonate)-17α de chloro-21 fluoro-9α méthyl-16β pregnadiène-1,4 diol-11β,17α trione-3,6,20.

8. (Propyl carbonate)-17α de fluoro-9α méthyl-16β pregnadiène-1,4 triol-11β,17α,21 trione-3,6,20.

9. (Propyl carbonate)-17α de chloro-21 fluoro-9α méthyl-16β pregnadiène-1,4 diol-11β,17α trione-3,6,20.

10. (Méthyl carbonate)-17α de fluoro-9α méthyl-16α pregnadiène-1,4 triol-11β,17α,21 trione-3,6,20.

11. (Méthyl carbonate)-17α de chloro-21 fluoro-9α méthyl-16α pregnadiène-1,4 diol-11β,17α trione-3,6,20.

12. (β-méthoxycarbonyl propionate)-17α de pregnène-4 triol-11β,17α,21 dione-3,20.

13. (β-méthoxycarbonyl propionate)-17α de fluoro-9α méthyl-16β pregnadiène-1,4 triol-11β,17α,21 dione-3,20.

14. (β-méthoxycarbonyl propionate)-17α de fluoro-9α méthyl-16β pregnadiène-1,4 triol-11β,17α,21 trione-3,6,20.

15. (β-méthoxycarbonyl propionate)-17α de chloro-21 fluoro-9α méthyl-16β pregnadiène-1,4 diol-11β,17α dione-3,20.

16. (β-méthoxycarbonyl propionate)-17α de chloro-21 fluoro-9α méthyl-16β pregnadiène-1,4 diol-11β,17α trione-3,6,20.

17. (β-méthoxycarbonyl propionate)-17α de chloro-21 fluoro-9α méthyl-16α pregnadiène-1,4 diol-11β,17α dione-3,20.

18. (γ-éthoxycarbonyl butyrate)-17α de chloro-21 fluoro-9α méthyl-16β pregnadiène-1,4 diol-11β,17α dione-3,20.

19. (β-méthoxycarbonyl propionate)-17α propionate-21 de fluoro-9α méthyl-16β pregnadiène-1,4 triol-11β,17α,21 dione-3,20.

20. (β-éthoxycarbonyl propionate)-17α de chloro-21 fluoro-9α méthyl-16β pregnadiène-1,4 diol-11β,17α dione-3,20.

21. Procédé de fabrication des corticoïdes selon la revendication 1, caractérisé par le fait que l'on fait réagir un composé représenté pr la formule générale (IV):

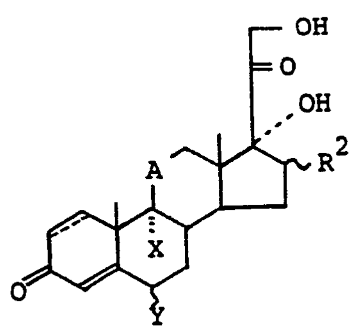

(IV)

(dans laquelle:

A, X, Y et $R^2$ sont tels que définis à la revendication 1)

avec un orthoester représenté par la formule générale (V):

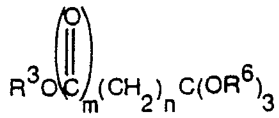

(V)

(dans laquelle:

m, n et $R^3$ sont tels que définis à la revendication 1; et

$R^6$ est un groupe alkyle ayant de 1 à 10 atomes de carbone,

à la condition que, si m vaut 0, n vaut O, et si m vaut l, n vaut 0 ou un nombre entier de 2 à 5), afin d'obtenir un composé représenté par la formule générale (VI):

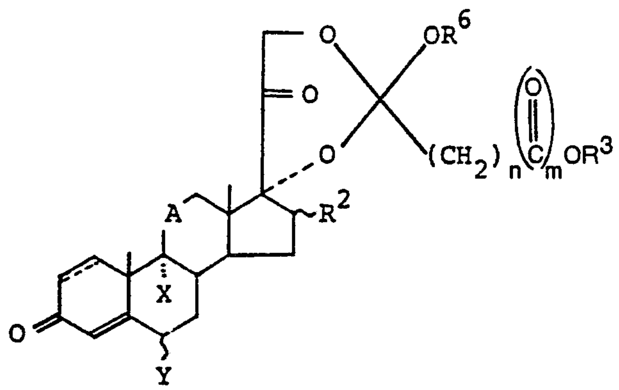

(VI)

(dans laquelle A, X, Y, m, n, $R^2$, $R^3$ et $R^6$ sont tels que définis ci-dessus), et que l'on hydrolyse ledit composé représenté par la formule générale (VI), afin d'obtenir un composé représenté par la formule générale (Ia):

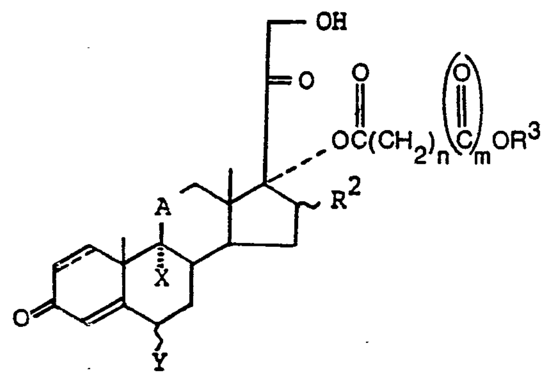

(Ia)

(dans laquelle A, X, Y, $R^2$, m, n et $R^3$ ont la même signification que celle indiquée ci-dessus), et, si on le désire, on fait réagir ledit composé représenté par la formule générale (Ia),

a) soit avec un acide sulfonique représenté par la formule générale (IIa) ou (IIb):

$$(R^4SO_2)_2O \qquad (IIa)$$

$$R^4SO_2X^1 \qquad (IIb)$$

(dans laquelle:

$R^4$ est tel que défini à la revendication 1; et

$X^1$ est un atome d'halogène),

afin d'obtenir un composé représenté par la formule générale (I), dans laquelle $R^1$ est un groupe représenté par la formule (II) telle que définie ci-dessus;

b) soit avec un acide carboxylique représenté par la formule générale (IIIa) ou (IIIb):

$$(R^5CO)_2O \qquad (IIIa)$$

$$R^5COX^1 \qquad (IIIb)$$

(dans laquelle:

$R^5$ est tel que défini à la revendication 1; et

$X^1$ est un atome d'halogène),

afin d'obtenir un composé représenté par la formule générale (I), dans laquelle $R^1$ est un groupe représenté par la formule (III) telle que définie ci-dessus;

c) soit à faire réagir le composé représenté par la formule générale (I) dans laquelle $R^1$ est un groupe représenté par la formule (II) (telle que définie ci-dessus), tel qu'obtenu par la réaction a) ci-dessus, avec un donneur d'ions halogène, afin d'obtenir un composé représenté par la formule générale (I), dans laquelle $R^1$ est un atome d'halogène.

22. Procédé de fabrication d'ortho esters cycliques-17α,21 corticoïdes représentés par la formule (VI):

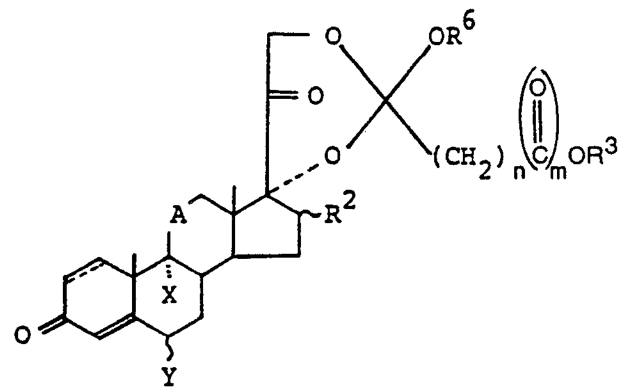

(VI)

(dans laquelle:

A, X, Y, m, n, $R^2$ et $R^3$ sont tels que définis à la revendication 1;

$R^6$ est un groupe alkyle ayant de 1 à 10 atomes de carbone; et

la liaison entre $C_1$ et $C_2$ est une simple liaison ou une double liaison),

qui consiste a faire rèagir un composé représenté par la formule générale (IV):

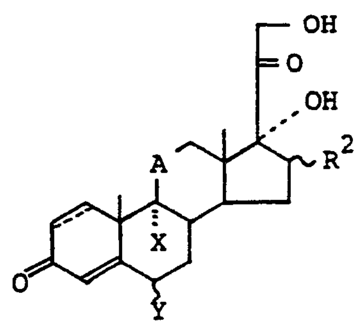

(IV)

[dans laquelle A, X, Y et $R^2$ ont la même signification que celle indiquée pour la formule générale (VI)], avec un orthoester représenté par la formule générale (V):

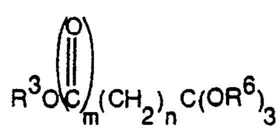

(V)

[dans laquelle m, n, $R^3$ et $R^6$ ont la même signification que celle indiquée pour la formule générale (VI), à la condition que, si m vaut 0, n vaut 0, et si m vaut 1, n vaut 0 ou un nombre entier de 2 à 5].

23. Procédé de fabrication de corticoïdes représentés par la formule générale (Ia):

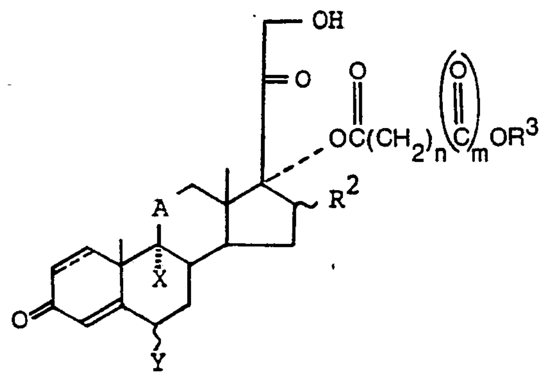

(Ia)

(dans laquelle:
A, X, Y, m, n, $R^2$ et $R^3$ sont tels que définis à la revendication 1; et
la liaison entre $C_1$ et $C_2$ est une simple liaison ou une double liaison),
qui consiste à hydrolyser un composé représenté par la formule générale (VI):

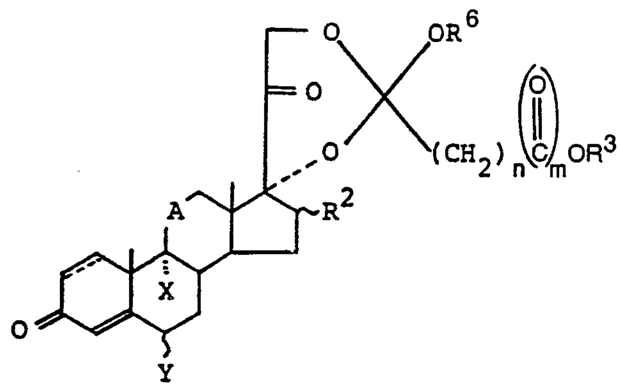

(VI)

(dans laquelle:
A, X, Y, n, m, $R^2$ et $R^3$ ont la même signification que celle indiquée pour la formule générale (Ia); et
$R^6$ est un groupe alkyle ayant de 1 à 10 atomes de carbone).

24. Procédé de fabrication de corticoïdes représentés par la formule générale (Ib):

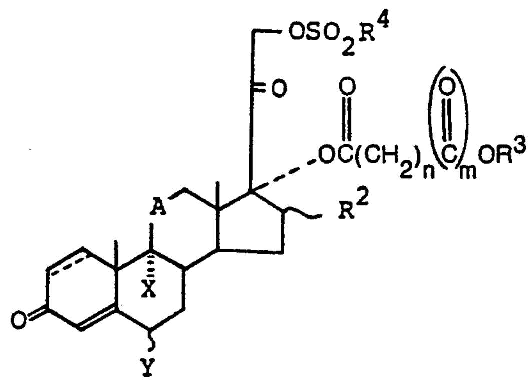

(Ib)

(dans laquelle:
A, X, Y, m, n, $R^2$, $R^3$ et $R^4$ sont tels que définis à la revendication 1; et
la liaison entre $C_1$ et $C_2$ est une simple liaison ou une double liaison),
qui consiste à faire réagir un composé représenté par la formule générale (Ia):

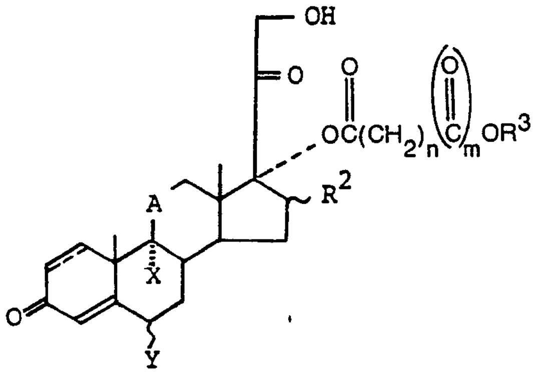

(Ia)

(dans laquelle A, X, Y, $R^2$, m, n et $R^3$ ont la même signification que celle indiquée pour la formule générale (Ib)),
avec un acide sulfonique représenté par la formule générale (IIa) ou (IIb):

$$(R^4SO_2)_2O \qquad (IIa)$$

$$R^4SO_2X^1 \qquad (IIb)$$

(dans laquelle:
$R^4$ a la même signification que celle indiquée pour la formule générale (Ib); et
$X^1$ est un atome d'halogène).

25. Procédé de fabrication de corticoïdes représentés par la formule générale (Ic):

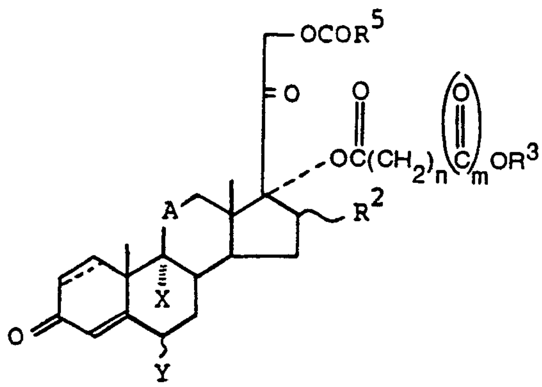

(Ic)

(dans laquelle:
A, X, Y, m, n, $R^2$, $R^3$ et $R^5$ sont tels que définis à la revendication 1; et
la liaison entre $C_1$ et $C_2$ est une simple liaison ou une double liaison),
qui consiste à faire réagir un composé représenté par la formule générale (Ia):

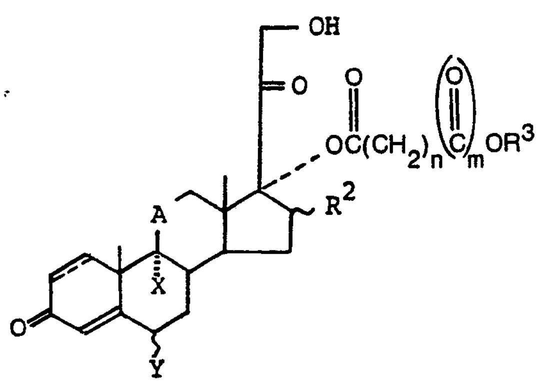

(Ia)

(dans laquelle A, X, Y, $R^2$, m, n et $R^3$ ont la même signification que celle indiquée pour la formule générale (Ic)),

avec un acide carboxylique représenté par la formule générale (IIIa) ou (IIIb):

$$(R^5CO)_2O \qquad (IIIa)$$

$$R^5COX^1 \qquad (IIIb)$$

(dans laquelle:

$R^5$ a la même signification que celle indiquée pour la formule générale (Ic); et

$X^1$ est un atome d'halogène).

26. Procédé de fabrication de corticoïdes représentés par la formule générale (Id):

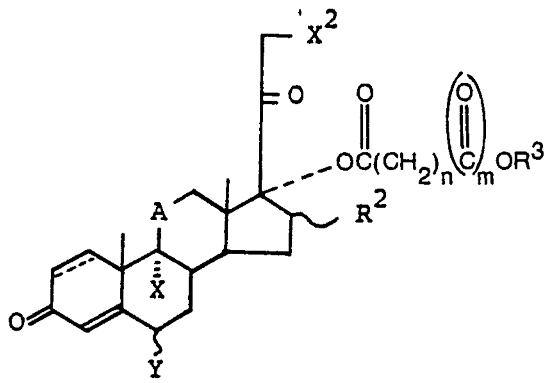

(Id)

(dans laquelle:

A, X, Y, m, n, $R^2$ et $R^3$ sont tels que définis à la revendication 1;

$X^2$ est un atome d'halogène; et

la liaison entre $C_1$ et $C_2$ est une simple liaison ou une double liaison),

qui consiste à faire réagir un composé représenté par la formule générale (Ib):

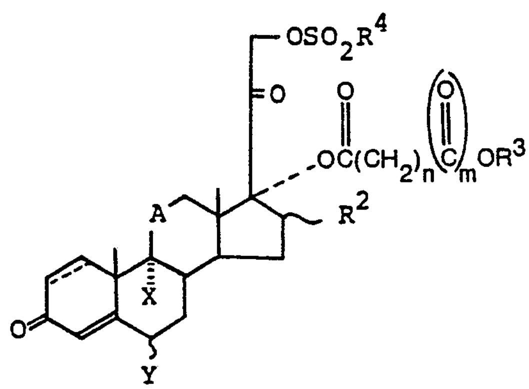

(Ib)

[dans laquelle:

A, X, Y, $R^2$, m, n et $R^3$ ont la même signification que celle indiquée pour la formule générale (Id); et

$R^4$ est un groupe alkyle ou un groupe alkyle halogéné ayant de 1 à 10 atomes de carbone]

avec un donneur d'ions halogène.

27. Procédé de fabrication de corticoïdes représentés par la formule générale (Id)':

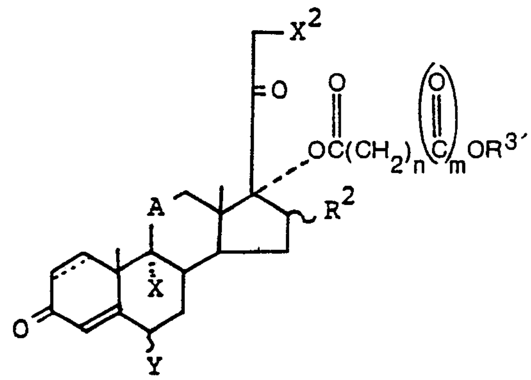

(Id)'

(dans laquelle:
A, X, Y, m, n et $R^3$ sont tels que définis à la revendication 1;
$X^2$ est un atome d'halogène;
$R^{3'}$ est un groupe alkyle ayant de 1 à 10 atomes de carbone; et
la liaison entre $C_1$ et $C_2$ est une simple liaison ou une double liaison),
qui consiste à faire réagir un composé représenté par la formule générale (Id):

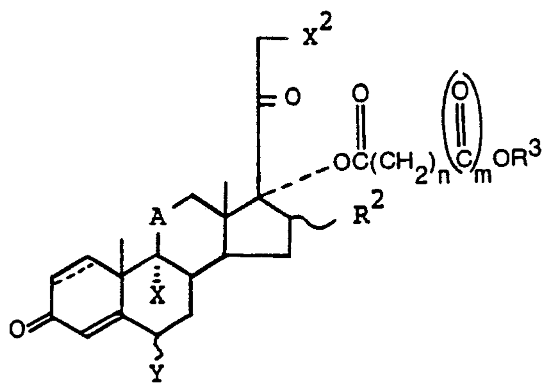

(Id)

[dans laquelle:
A, X, Y, $R^2$, m, n et $X^2$ ont la même signification que celle indiquée pour la formule générale (Id)'; et
$R^3$ est un groupe alkyle ayant de 1 à 10 atomes de carbone, mais est différent de $R^{3'}$ de la formule générale (Id)']
avec un alcool représenté par la formule générale (VIII):

$$R^{3'}—OH \qquad (VIII)$$

[dans laquelle $R^{3'}$ a la même signification que celle indiquée pour la formule générale (Id)']
afin d'effectuer une trans-estérification.

28. Composition pharmaceutique comprenant une quantité efficace de corticoïdes tels que définis à la revendication 1.